# EUROPEAN PATENT APPLICATION

(11) **EP 4 778 535 A1**
(43) Date of publication of application: **22.07.2026**
(21) Application number: 24864771.1
(22) Date of filing: 14.09.2024
(51) Int. Cl.: A61K 39/395, C07K 16/28, C07K 16/46

(54) **PHARMACEUTICAL COMBINATION AND USE**

(30) Priority: 14.09.2023 CN 202311192415
(71) Applicant: Akeso Biopharma Co., Ltd., Zhongshan, Guangdong 528437 (CN)
(72) Inventor: WANG, Zhongmin, Zhongshan, Guangdong 528437 (CN); LI, Baiyong, Zhongshan, Guangdong 528437 (CN); XIA, Yu, Zhongshan, Guangdong 528437 (CN)
(74) Representative: Berggren Oy
(86) International application number: PCT/CN2024/119075
(87) International publication number: WO 2025/056056

(57) **Abstract**

Provided are a pharmaceutical combination and a use. The pharmaceutical combination contains a bispecific protein specifically binding to PD-1 and VEGFA, and a bispecific protein specifically binding to TIGIT and TGF-βR. The pharmaceutical combination can effectively treat or prevent tumors, and has good application prospects.

## Description

### CROSS-REFERENCE TO RELATED APPLICATIONS

The present application is based on Chinese Patent Application No. 202311192415.9 filed on September 14, 2023 and claims priority thereto, which is incorporated herein by reference in its entirety.

### TECHNICAL FIELD

The present disclosure belongs to biopharmaceuticals, and relates to a pharmaceutical combination and use thereof. Specifically, the pharmaceutical combination comprises a bispecific protein specifically binding to PD-1 and VEGFA and a bispecific protein specifically binding to TIGIT and TGF-βR. The pharmaceutical combination may be in the form of a pharmaceutical composition or a pharmaceutical combination product.

### BACKGROUND

The transmembrane receptor PD-1 (programmed cell death protein-1) is a member of the CD28 gene family, and is expressed on activated T cells, B cells, and myeloid cells. Ligands of PD-1, PDL1 (programmed cell death 1 ligand 1, also abbreviated as PD-L1) and PDL2 (programmed cell death 1 ligand 2, also abbreviated as PD-L2), are both members of the B7 superfamily. PDL1 is expressed on a variety of cells including T cells, B cells, endothelial cells and epithelial cells, while PDL2 is expressed only on antigen-presenting cells such as dendritic cells and macrophages. The PD-1/PDL1 signaling pathway plays an important role in regulating immune tolerance, microbial infection, and tumor immune escape. PD-1 is mainly expressed in immune cells such as T cells, and the ligand PDL1 of PD-1 is highly expressed in a plurality of human tumor tissues. Blocking the PD-1/PDL1 signaling pathway can activate inhibited T cells, which in turn attack cancer cells. Blocking the PD-1/PDL1 signaling can promote the proliferation of tumor antigen-specific T cells, play a role in killing tumor cells, and further inhibit local tumor growth (Julie R et al., 2012, N Engl J Med., 366:2455-2465). In addition, tumors with high PDL1 expression are associated with cancers that are difficult to detect (Hamanishi et al., 2007, Proc. Natl. Acad. Sci. USA, 104: 3360-5). An effective method is the *in-vivo* injection of an anti-PD-1 antibody to modulate the expression of PD-1. Due to the broad-spectrum anti-tumor prospects and surprising efficacy of PD-1 antibodies, it is widely accepted in the industry that antibodies targeting the PD-1 pathway will bring about breakthroughs in the treatment of various tumors, for example, non-small cell lung cancer, renal cell carcinoma, ovarian cancer, melanoma (Homet M. B., Parisi G., et al., 2015, Semin Oncol., 42(3): 466-473), leukemia and anemia (Held SA, Heine A, et al., 2013, Curr Cancer Drug Targets., 13(7): 768-74).

Vascular endothelial growth factor (VEGF) is a growth factor that can promote division and proliferation of endothelial cells, promote formation of new blood vessels and improve blood vessel permeability. It binds to vascular endothelial growth factor receptors on the cell surface and plays a role by activating tyrosine kinase signal transduction pathways. In tumor tissues, tumor cells, and macrophages and mast cells invading into tumors can secrete high-level VEGF, stimulate tumor vascular endothelial cells in a paracrine form, promote proliferation and migration of endothelial cells, induce angiogenesis, promote continuous growth of tumor, improve vascular permeability, cause fibrin deposition in surrounding tissues, and promote infiltration of mononuclear cells, fibroblasts and endothelial cells, which facilitates the formation of tumor stroma and entry of tumor cells into new blood vessels, and promote tumor metastasis. Therefore, inhibiting tumor angiogenesis is considered as one of the most promising tumor treatment methods at present. The VEGF family includes: VEGFA, VEGFB, VEGFC, VEGFD, and PIGF. Vascular endothelial growth factor receptors (VEGFRs) include VEGFR1 (also known as Flt1), VEGFR2 (also known as KDR or Flk1), VEGFR3 (also known as Flt4), and Neuropilin-1 (NRP-1). The first three receptors are members of the tyrosine kinase superfamily, and are of similar structures composed of an extramembrane region, a transmembrane segment and an intramembrane region, where the extramembrane region is composed of an immunoglobulin-like domain, and the intramembrane region is a tyrosine kinase region. VEGFR1 and VEGFR2 are mainly found on the surface of vascular endothelial cells, and VEGFR3 is mainly found on the surface of lymphatic endothelial cells.

Molecules of the VEGF family have different affinities for these receptors. VEGFA mainly acts in combination with VEGFR1, VEGFR2, and NRP-1. VEGFR1 is the first identified receptor, and has a higher affinity for VEGFA than VEGFR2 under normal physiological conditions but a lower tyrosinase activity in the intracellular segment than VEGFR2 (Ma Li, Chinese Journal of Birth Health and Heredity, 24(5) (2016): 146-148). VEGFR2 is the primary regulator of angiogenesis and vascular engineering, and has a much higher tyrosine kinase activity than VEGFR1. VEGFR2, after binding to ligand VEGFA, mediates the proliferation, differentiation and other progresses of vascular endothelial cells, as well as the formation process of blood vessels and the permeability of blood vessels (Roskoski R Jr. et al., Crit Rev Oncol Hematol, 62(3) (2007): 179-213). VEGFA, after binding to VEGFR2, mediates the transcription and expression of related intracellular protein genes through the downstream PLC-γ-PKC-Raf-MEK-MAPK signaling pathway, and thus promotes the proliferation of vascular endothelial cells (Takahashi T et al., Oncogene, 18(13) (1999): 2221-2230).

VEGFR3 is one of the tyrosine kinase family members, and is mainly expressed in embryonic vascular endothelial cells and mature lymphatic endothelial cells, and VEGFC and VEGFD bind to VEGFR3 to stimulate proliferation and migration of lymphatic endothelial cells and promote neogenesis of lymphatic vessels; NRP-1 is a non-tyrosine kinase transmembrane protein, and is incapable of independently transducing biological signals but able to mediate signaling only after forming a complex with a VEGF tyrosine kinase receptor. (Ma Li, Chinese Journal of Birth Health and Heredity, 24(5) (2016): 146-148).

VEGFA and VEGFR2 are mainly involved in regulation of angiogenesis, where before and after the binding of VEGFA to VEGFR2, a cascade reaction of numerous intermediate signals in upstream and downstream pathways is formed, and finally the physiological functions are changed by proliferation, survival, migration, permeability increase, infiltration to peripheral tissues and other patterns of endothelial cells (Dong Hongchao et al., Sep. 2014, Journal of Modern Oncology, 22(9): 2231-3). TIGIT (T cell Ig and ITIM domain, also known as WUCAM, Vstm3, or VSIG9) is a member of the poliovirus receptor (PVR)/Nectin family. TIGIT consists of an extracellular immunoglobulin variable region (IgV) domain, a type I transmembrane domain, and an intracellular domain with a classical immunoreceptor tyrosine-based inhibitory motif (ITIM), and an immunoglobulin tail tyrosine (ITT) motif. TIGIT is highly expressed in lymphocytes, especially in effector and regulatory CD4⁺ T cells (Treg cells), follicular helper CD4⁺ T cells, effector CD8⁺ T cells, and natural killer (NK) cells (Yu X, Harden K, Gonzalez L C, et al., The surface protein TIGIT suppresses T cell activation by promoting the generation of mature immunoregulatory dendritic cells [J]. Nature immunology, 2009, 10(1): 48).

CD155 (also known as PVR, Necl5, or Tage4), CD112 (also known as PVRL2/nectin 2), and CD113 (also known as PVRL3) are ligands to which TIGIT binds (Martinet L, Smyth M J., Balancing natural killer cell activation through paired receptors [J]. Nature Reviews Immunology, 2015, 15(4): 243-254). CD155 is a high-affinity ligand for TIGIT. In NK cells, TIGIT binding to the ligands CD155 and CD112 can inhibit the killing effect of NK cells on cells with high CD155 and CD112 expression (Stanietsky N, Simic H, Arapovic J, et al., The interaction of TIGIT with PVR and PVRL2 inhibits human NK cell cytotoxicity [J]. Proceedings of the National Academy of Sciences, 2009, 106(42): 17858-17863). Moreover, studies have reported that the killing effect of CD8⁺ T cells can be enhanced when PD-1 and TIGIT are blocked simultaneously (Johnston R J, Comps-Agrar L, Hackney J, et al., The immunoreceptor TIGIT regulates anti-tumor and antiviral CD8+ T cell effector function [J]. Cancer cell, 2014, 26(6): 923-937). Recent studies have shown that TIGIT, as an immune checkpoint of NK cells, can cause NK cell exhaustion during tumor progression and have demonstrated that anti-TIGIT monoclonal antibodies can reverse NK cell exhaustion and be used for immunotherapy of a variety of tumors such as non-small cell lung cancer, small cell lung cancer, breast cancer, ovarian cancer, colorectal cancer, melanoma, pancreatic cancer, cervical tumor, multiple myeloma, non-Hodgkin's lymphoma, B-lymphoma, and plasma cell cancer (Zhang Q, Bi J, Zheng X, et al., Blockade of the checkpoint receptor TIGIT prevents NK cell exhaustion and elicits potent anti-tumor immunity [J]. Nature immunology, 2018, 19(7):723-732).

The transforming growth factor-β (TGF-β) superfamily is a class of functionally diverse cytokines and is further divided into subfamilies such as TGF-β, activins, inhibins, growth differentiation factors (GDFs), glial cell line-derived neurotrophic factors (GDNFs), Nodal, Lefty, and anti-Müllerian hormone (Table A). The three proteins of the TGF-β subfamily are known as TGF-β1, TGF-β2, and TGF-β3, respectively, among which TGF-β1 is the most highly expressed subtype. TGF-β1, TGF-β2, and TGF-β3, upon binding to receptors, mediate a range of biological reactions through the Smad and non-Smad signaling pathways, including promotion of epithelial-mesenchymal transitions (EMTs), promotion of tissue fibrosis, promotion of angiogenesis, promotion of tumor immune escape, cancer inhibition and promotion dual effects, etc. (J Massagué. TGFbeta in Cancer [J]. Cell, 2008, 134(2):215-230).

TGF-β receptors (TGF-βRs) are widely distributed on the surface of normal and tumor cells in humans and include three receptor superfamilies: the classical TGF-β receptor family type I includes ALK1-7, and TGF-βRI (also written as TPRI) is also known as ALK5; the classical TGF-β receptor family type II includes TGF-βRII (also written as TβRII), ActRII, ActRIIB, AMHRII, and BMPRII; the TGF-β receptor superfamily type III includes Betaglycan (also known as TGF-βRIII) and Endoglin. TGF-βRI, TGF-βRII, and TGF-βRIII are each able to bind to TGF-β1, TGF-β2, and TGF-β3 (Pawlak John B, Blobe Gerard C, TGF-β superfamily co-receptors in cancer. DevDyn, 2021). The TGF-β family and receptors are shown in Table A (summarized based on the review Carl-Henrik Heldin1 and Aristidis Moustakas. Cold Spring Harb Perspect Biol. 2016). TGF-βRI and TGF-βRII are serine/threonine protein kinase receptors. TGF-βRII, as a key molecule of signal transduction in the TGF-β signaling pathways, can bind to TGF-β with a high affinity and then form a heterotetramer receptor complex with a TGF-βRI dimer. Through the self-phosphorylation of TGF-βRII, TGF-βRI is further phosphorylated and activated. The activated TGF-βRI phosphorylates downstream Smad pathway-related proteins and regulates the transcription and translation of downstream target genes, thereby causing disease-related biological reactions. TGF-βRIII has a weaker affinity for TGF-β than TGF-βRI and TGF-βRII and has no intracellular segment. Thus, it cannot be connected to downstream signaling pathways. Its function is to capture and present TGF-β to TGF-βRII. Other TGF-β receptors can also bind to TGF-β (Sang, X.H., et al., Advances in research on small-molecule inhibitors targeting TGF-β and receptors [J]. Journal of Pharmacy, 2019(9*)).*

For many cancer patients, the disease is still uncontrollable for a long term after surgery or chemotherapeutic drug treatment, and the 5-year survival rate is still very low. In summary, developing a treatment means or combination administration regimen with lower toxicity and higher efficacy is clinically of great meaning.

### SUMMARY

After intensive studies and creative efforts, the inventors have surprisingly found that combining a bispecific protein specifically binding to PD-1 and VEGFA with a bispecific protein specifically binding to TIGIT and TGF-βR has a pharmacological effect of effectively inhibiting tumor growth, which is superior to that of either the bispecific protein specifically binding to PD-1 and VEGFA or the bispecific protein specifically binding to TIGIT and TGF-βR alone. The present disclosure is detailed below:
One aspect of the present disclosure relates to a pharmaceutical combination comprising an effective amount of a first bispecific protein and an effective amount of a second bispecific protein, wherein:
(1) the first bispecific protein comprises:
   a first protein functional region specifically binding to PD-1, and
   a second protein functional region specifically binding to VEGFA,
   wherein
   the first protein functional region comprises a heavy chain variable region VH1 and a light chain variable region VL1, wherein the VH1 comprises an HCDR1 set forth in SEQ ID NO: 1, an HCDR2 set forth in SEQ ID NO: 2, and an HCDR3 set forth in SEQ ID NO: 3; and the VL1 comprises an LCDR1 set forth in SEQ ID NO: 4, an LCDR2 set forth in SEQ ID NO: 5, and an LCDR3 set forth in SEQ ID NO: 6;
   the second protein functional region comprises a heavy chain variable region VH2 and a light chain variable region VL2, wherein the VH2 comprises an HCDR1 set forth in SEQ ID NO: 7, an HCDR2 set forth in SEQ ID NO: 8, and an HCDR3 set forth in SEQ ID NO: 9; and the VL2 comprises an LCDR1 set forth in SEQ ID NO: 10, an LCDR2 set forth in SEQ ID NO: 11, and an LCDR3 set forth in SEQ ID NO: 12;
(2) the second bispecific protein comprises:
   a third protein functional region specifically binding to TIGIT, and
   a fourth protein functional region specifically binding to TGF-β,
   wherein
   the third protein functional region comprises a heavy chain variable region VH3 and a light chain variable region VL3, wherein the VH3 comprises an HCDR1 set forth in SEQ ID NO: 13, an HCDR2 set forth in SEQ ID NO: 14, and an HCDR3 set forth in SEQ ID NO: 15; and the VL3 comprises an LCDR1 set forth in SEQ ID NO: 16, an LCDR2 set forth in SEQ ID NO: 17, and an LCDR3 set forth in SEQ ID NO: 18;
   the fourth protein functional region is a TGF-β receptor, an extracellular region of a TGF-β receptor, a truncated fragment of an extracellular region of a TGF-β receptor with TGF-β receptor function, or a variant of an extracellular region of a TGF-β receptor with TGF-β receptor function.

In the present disclosure, the bispecific protein may also be known as a bispecific molecule. The first bispecific protein may also be referred to as a first bispecific molecule. The second bispecific protein may also be referred to as a second bispecific molecule.

In some embodiments of the present disclosure, the bispecific protein is a bispecific antibody. In some embodiments of the present disclosure, the first bispecific protein is a bispecific antibody, preferably an anti-PD-1/anti-VEGFA bispecific antibody.

In some embodiments of the present disclosure, the bispecific protein is an antibody-receptor fusion protein. In some embodiments of the present disclosure, the second bispecific protein is an antibody-receptor fusion protein, preferably an anti-TIGIT antibody-TGF-βR fusion protein.

In some embodiments of the present disclosure, provided is the pharmaceutical combination, wherein
the heavy chain variable region VH1 comprises an amino acid sequence selected from SEQ ID NO: 20 and SEQ ID NO: 24; and
the light chain variable region VL1 comprises an amino acid sequence selected from SEQ ID NO: 22, SEQ ID NO: 26, and SEQ ID NO: 35.

In some embodiments of the present disclosure, provided is the pharmaceutical combination, wherein
the heavy chain variable region VH1 comprises an amino acid sequence set forth in SEQ ID NO: 20, and the light chain variable region VL1 comprises an amino acid sequence set forth in SEQ ID NO: 22;
the heavy chain variable region VH1 comprises an amino acid sequence set forth in SEQ ID NO: 20, and the light chain variable region VL1 comprises an amino acid sequence set forth in SEQ ID NO: 26;
the heavy chain variable region VH1 comprises an amino acid sequence set forth in SEQ ID NO: 20, and the light chain variable region VL1 comprises an amino acid sequence set forth in SEQ ID NO: 35;
the heavy chain variable region VH1 comprises an amino acid sequence set forth in SEQ ID NO: 24, and the light chain variable region VL1 comprises an amino acid sequence set forth in SEQ ID NO: 22;
the heavy chain variable region VH1 comprises an amino acid sequence set forth in SEQ ID NO: 24, and the light chain variable region VL1 comprises an amino acid sequence set forth in SEQ ID NO: 26; or
the heavy chain variable region VH1 comprises an amino acid sequence set forth in SEQ ID NO: 24, and the light chain variable region VL1 comprises an amino acid sequence set forth in SEQ ID NO: 35.

In some embodiments of the present disclosure, provided is the pharmaceutical combination, wherein
the heavy chain variable region VH2 comprises an amino acid sequence set forth in SEQ ID NO: 31, and the light chain variable region VL2 comprises an amino acid sequence set forth in SEQ ID NO: 33.

In some embodiments of the present disclosure, provided is the pharmaceutical combination, wherein
the heavy chain variable region VH3 comprises an amino acid sequence selected from SEQ ID NO: 46, SEQ ID NO: 50, SEQ ID NO: 52, SEQ ID NO: 54, and SEQ ID NO: 56; and
the light chain variable region VL3 comprises an amino acid sequence selected from SEQ ID NO: 48, SEQ ID NO: 58, SEQ ID NO: 60, SEQ ID NO: 62, and SEQ ID NO: 64.

In some embodiments of the present disclosure, provided is the pharmaceutical combination, wherein
the heavy chain variable region VH3 comprises an amino acid sequence set forth in SEQ ID NO: 46, and the light chain variable region VL3 comprises an amino acid sequence set forth in SEQ ID NO: 48;
the heavy chain variable region VH3 comprises an amino acid sequence set forth in SEQ ID NO: 50, and the light chain variable region VL3 comprises an amino acid sequence set forth in SEQ ID NO: 58;
the heavy chain variable region VH3 comprises an amino acid sequence set forth in SEQ ID NO: 50, and the light chain variable region VL3 comprises an amino acid sequence set forth in SEQ ID NO: 64;
the heavy chain variable region VH3 comprises an amino acid sequence set forth in SEQ ID NO: 52, and the light chain variable region VL3 comprises an amino acid sequence set forth in SEQ ID NO: 60;
the heavy chain variable region VH3 comprises an amino acid sequence set forth in SEQ ID NO: 52, and the light chain variable region VL3 comprises an amino acid sequence set forth in SEQ ID NO: 62;
the heavy chain variable region VH3 comprises an amino acid sequence set forth in SEQ ID NO: 54, and the light chain variable region VL3 comprises an amino acid sequence set forth in SEQ ID NO: 60;
the heavy chain variable region VH3 comprises an amino acid sequence set forth in SEQ ID NO: 54, and the light chain variable region VL3 comprises an amino acid sequence set forth in SEQ ID NO: 62;
the heavy chain variable region VH3 comprises an amino acid sequence set forth in SEQ ID NO: 56, and the light chain variable region VL3 comprises an amino acid sequence set forth in SEQ ID NO: 58; or
the heavy chain variable region VH3 comprises an amino acid sequence set forth in SEQ ID NO: 56, and the light chain variable region VL3 comprises an amino acid sequence set forth in SEQ ID NO: 64.

In some embodiments of the present disclosure, provided is the pharmaceutical combination, wherein
the fourth protein functional region is TGF-βRI, TGF-βRII, TGF-βRIII, an extracellular region of TGF-βRI, an extracellular region of TGF-βRII, an extracellular region of TGF-βRIII, a truncated fragment of an extracellular region of TGF-βRI with TGF-β receptor function, a truncated fragment of an extracellular region of TGF-βRII with a TGF-β receptor function, a truncated fragment of an extracellular region of TGF-βRIII with a TGF-β receptor function, or a variant of an extracellular region of TGF-β receptor with a TGF-β receptor function.

The variant of the extracellular region of the TGF-β receptor with TGF-β receptor function refers to a protein that retains TGF-β receptor function after one or several (e.g., 2, 3, 4, 5, or more) amino acid replacements, deletions, and/or insertions are made in the extracellular region of the TGF-β receptor.

In some embodiments of the present disclosure, provided is the pharmaceutical combination, wherein
the first protein functional region is IgG, and the second protein functional region is a single-chain variable fragment; or
the first protein functional region is a single-chain variable fragment, and the second protein functional region is IgG.

In some embodiments of the present disclosure, provided is the pharmaceutical combination, wherein
the first protein functional region is IgG1**,** and the second protein functional region is a single-chain variable fragment; or
the first protein functional region is a single-chain variable fragment, and the second protein functional region is IgG1.

In some embodiments of the present disclosure, provided is the pharmaceutical combination, wherein
the third protein functional region is IgG or a single-chain variable fragment.

In some embodiments of the present disclosure, provided is the pharmaceutical combination, wherein
the third protein functional region is IgG4 or a single-chain variable fragment.

In some embodiments of the present disclosure, provided is the pharmaceutical combination, wherein
the IgG is IgG1, IgG2, IgG3, or IgG4.

In some embodiments of the present disclosure, provided is the pharmaceutical combination, wherein
according to the EU numbering system, the heavy chain constant region of IgG1 has one of the following four combinations of mutations:
L234A and L235A;
L234A and G237A;
L235A and G237A; or
L234A, L235A, and G237A.

In some embodiments of the present disclosure, provided is the pharmaceutical combination, wherein
according to the EU numbering system, the heavy chain constant region of IgG4 has one of the following four combinations of mutations:
F234A and L235A;
F234A and G237A;
L235A and G237A; or
F234A, L235A, and G237A.

In some embodiments of the present disclosure, provided is the pharmaceutical combination, wherein
the second protein functional region is IgG, the heavy chain thereof comprises an amino acid sequence set forth in SEQ ID NO: 38, SEQ ID NO: 40, or SEQ ID NO: 42, and the light chain thereof comprises an amino acid sequence set forth in SEQ ID NO: 44.

In some embodiments of the present disclosure, provided is the pharmaceutical combination, wherein
the third protein functional region is IgG, the heavy chain comprises an amino acid sequence set forth in SEQ ID NO: 66 or SEQ ID NO: 70, and the light chain comprises an amino acid sequence set forth in SEQ ID NO: 68.

In some embodiments of the present disclosure, provided is the pharmaceutical combination, wherein
the fourth protein functional region comprises an amino acid sequence set forth in any one of SEQ ID NO: 72 to SEQ ID NO: 78 and SEQ ID NO: 88.

In some embodiments of the present disclosure, provided is the pharmaceutical combination, wherein
the first protein functional region and the second protein functional region are linked directly or via a linker;
the third protein functional region and the fourth protein functional region are linked directly or via a linker; and/or
a heavy chain variable region of a single-chain variable fragment and a light chain variable region of a single-chain variable fragment are linked directly or via a linker.

In some embodiments of the present disclosure, provided is the pharmaceutical combination, wherein
the linker is a polypeptide set forth in SEQ ID NO: 91 or SEQ ID NO: 92, or a polypeptide obtained by concatenating a plurality of (e.g., 2, 3, 4, 5, or 6) polypeptides set forth in SEQ ID NO: 91, or a polypeptide obtained by concatenating a plurality of (e.g., 2, 3, 4, or 5) polypeptides set forth in SEQ ID NO: 91 and further concatenating the polypeptides with a polypeptide set forth in SEQ ID NO: 92;
preferably, the linker comprises an amino acid sequence independently selected from SEQ ID NO: 36, SEQ ID NO: 37, and SEQ ID NO: 83.

In some embodiments of the present disclosure, provided is the pharmaceutical combination, wherein
the first protein functional region, the second protein functional region, the third protein functional region, and the fourth protein functional region are independently 1, 2 or more in number;
preferably, the first protein functional region is 2 in number, and the second protein functional region is 1 in number;
preferably, the third protein functional region is 1 in number, and the fourth protein functional region is 2 in number.

In some embodiments of the present disclosure, provided is the pharmaceutical combination, wherein
the second protein functional region is one IgG, the first protein functional region is two single-chain variable fragments, and the single-chain variable fragments are linked to the C-termini or the N-termini of the two heavy chains of the second protein functional region, respectively. In some embodiments of the present disclosure, provided is the pharmaceutical combination, wherein
the second protein functional region is one IgG1**,** the first protein functional region is two single-chain variable fragments, and the single-chain variable fragments are linked to the C-termini or the N-termini of the two heavy chains of the second protein functional region, respectively. In some embodiments of the present disclosure, provided is the pharmaceutical combination, wherein
the first protein functional region is one IgG, the second protein functional region is two single-chain variable fragments, and the single-chain variable fragments are linked to the C-termini or the N-termini of the two heavy chains of the first protein functional region, respectively.

In some embodiments of the present disclosure, provided is the pharmaceutical combination, wherein
the first protein functional region is one IgG1**,** the second protein functional region is two single-chain variable fragments, and the single-chain variable fragments are linked to the C-termini or the N-termini of the two heavy chains of the first protein functional region, respectively.

In one or more embodiments of the present disclosure, the bispecific protein is a bispecific antibody, which is in the form of IgG-scFv, i.e., the Morrison format.

In one or more embodiments of the present disclosure, for the bispecific antibody, the single-chain variable fragment is linked to the C terminus of the heavy chain of the immunoglobulin. Since an immunoglobulin has two heavy chains, two single-chain variable fragment molecules are linked to one immunoglobulin molecule. Preferably, the two single-chain variable fragment molecules are identical. Preferably, the single-chain variable fragment is linked to the C-terminus of the heavy chain of the immunoglobulin by forming an amide bond via the aforementioned linker.

In one or more embodiments of the present disclosure, the constant regions of the immunoglobulin are humanized. For example, the heavy chain constant regions are all Ig gamma-1 chain C regions, such as ACCESSION: P01857 or Ig gamma-4 chain C regions, such as ACCESSION: P01861.1; the light chain constant regions are all Ig kappa chain C regions, such as ACCESSION: P01834.

In some embodiments of the present disclosure, provided is the pharmaceutical combination, wherein
the third protein functional region is one IgG, the fourth protein functional region is 2 in number, and the fourth protein functional regions are linked to the C-termini or the N-termini of the two heavy chains of the third protein functional region, respectively.

In some embodiments of the present disclosure, provided is the pharmaceutical combination, wherein
the third protein functional region is one IgG4, the fourth protein functional region is 2 in number, and the fourth protein functional regions are linked to the C-termini or the N-termini of the two heavy chains of the third protein functional region, respectively. In some embodiments of the present disclosure, provided is the pharmaceutical combination, wherein
the first bispecific protein comprises:
   a first protein functional region specifically binding to PD-1, and
   a second protein functional region specifically binding to VEGFA;
   the first protein functional region is 2 in number, and the second protein functional region is 1 in number,
   wherein the second protein functional region is IgG, and the first protein functional region is a single-chain variable fragment;
   the IgG comprises a heavy chain having an amino acid sequence set forth in SEQ ID NO: 42, and a light chain having an amino acid sequence set forth in SEQ ID NO: 44;
   the single-chain variable fragment comprises a heavy chain variable region having an amino acid sequence set forth in SEQ ID NO: 24, and a light chain variable region having an amino acid sequence set forth in SEQ ID NO: 35;
   two single-chain variable fragments are linked to the C-termini of the two heavy chains of the IgG, respectively;
   the first protein functional region and the second protein functional region are linked via a first linker; the heavy chain variable region of the single-chain variable fragment and the light chain variable region of the single-chain variable fragment are linked via a second linker; the first linker and the second linker are identical or different;
   preferably, the first linker and the second linker each comprise an amino acid sequence independently selected from SEQ ID NO: 36 and SEQ ID NO: 37;
   preferably, the first linker and the second linker both comprise an amino acid sequence set forth in SEQ ID NO: 36.

In some embodiments of the present disclosure, provided is the pharmaceutical combination, wherein
the second bispecific protein comprises:
a third protein functional region specifically binding to TIGIT, and
a fourth protein functional region specifically binding to TGF-β,
wherein
the third protein functional region is 1 in number, and the fourth protein functional region is 2 in number;
the third protein functional region is IgG, and the fourth protein functional region is a truncated fragment of an extracellular region of a TGF-β receptor with TGF-β receptor function;
the IgG comprises a heavy chain having an amino acid sequence set forth in SEQ ID NO: 66 or SEQ ID NO: 70, and a light chain having an amino acid sequence set forth in SEQ ID NO: 68;
the fourth protein functional region comprises an amino acid sequence set forth in SEQ ID NO: 72 or SEQ ID NO: 88;
the fourth protein functional regions are linked to the C-termini of the two heavy chains of the IgG via a third linker;
preferably, the third linker comprises an amino acid sequence set forth in SEQ ID NO: 83.

In some embodiments of the present disclosure, provided is the pharmaceutical combination, wherein the first bispecific protein is selected from the anti-PD-1-anti-VEGFA bispecific antibodies VP101(M), VP101(G4M), and VP101(hG1DM) of the present disclosure, and the second bispecific protein is selected from the anti-TIGIT antibody-TGF-βR fusion proteins TF01, TF02, and TF01T of the present disclosure.

In some embodiments of the present disclosure, provided is the pharmaceutical combination, wherein the first bispecific protein is the anti-PD-1-anti-VEGFA bispecific antibody VP101(M) of the present disclosure, and the second bispecific protein is selected from the anti-TIGIT antibody-TGF-βR fusion proteins TF01, TF02, and TF01T of the present disclosure.

In some embodiments of the present disclosure, provided is the pharmaceutical combination, wherein the first bispecific protein is the anti-PD-1-anti-VEGFA bispecific antibody VP101(G4M) of the present disclosure, and the second bispecific protein is selected from the anti-TIGIT antibody-TGF-βR fusion proteins TF01, TF02, and TF01T of the present disclosure.

In some embodiments of the present disclosure, provided is the pharmaceutical combination, wherein the first bispecific protein is the anti-PD-1-anti-VEGFA bispecific antibody VP101(hG1DM) of the present disclosure, and the second bispecific protein is selected from the anti-TIGIT antibody-TGF-βR fusion proteins TF01, TF02, and TF01T of the present disclosure.

In some embodiments of the present disclosure, provided is the pharmaceutical combination, wherein the first bispecific protein is the anti-PD-1-anti-VEGFA bispecific antibody VP101(hG1DM) of the present disclosure, and the second bispecific protein is the anti-TIGIT antibody-TGF-βR fusion protein TF01T of the present disclosure.

In some embodiments of the present disclosure, provided is the pharmaceutical combination, wherein the first bispecific protein and the second bispecific protein are mixed together, and the pharmaceutical combination is a pharmaceutical composition.

In some embodiments of the present disclosure, the provided is the pharmaceutical combination, wherein the first bispecific protein and the second bispecific protein are in separate packages, and the pharmaceutical combination is a combination product.

In some embodiments of the present disclosure, provided is the pharmaceutical combination, wherein a mass ratio of the first bispecific protein to the second bispecific protein is (10:1) to (1:5), preferably (5:1) to (1:3) or (3:1) to (1:2), and more preferably 2:1.

In some embodiments of the present disclosure, provided is the pharmaceutical combination, wherein the first bispecific protein is the anti-PD-1/anti-VEGFA bispecific antibody VP101(hG1DM) of the present disclosure, the second bispecific protein is the anti-TIGIT antibody-TGF-βR fusion protein TF01T of the present disclosure, and a mass ratio of the first bispecific protein to the second bispecific protein is (10:1) to (1:5), preferably (5:1) to (1:3) or (3:1) to (1:2), and more preferably 2:1.

In some embodiments of the present disclosure, provided is the pharmaceutical combination, wherein unit doses of the first bispecific protein and the second bispecific protein are independently 100 mg to 2500 mg, 100 mg to 2000 mg, 100 mg to 1500 mg, 100 mg to 1200 mg, 100 mg to 1000 mg, 200 mg to 800 mg, 200 mg to 500 mg, 300 mg to 600 mg, 400 mg to 500 mg, or 450 mg, based on the mass of the first bispecific protein or the second bispecific protein.

In some embodiments of the present disclosure, provided is the pharmaceutical combination, which further comprises an effective amount of an anti-tumor chemotherapeutic drug, such as a platinum-based drug, an alkylating agent, an antimetabolite, an anti-tumor antibiotic, a plant-based anticancer agent, a hormone, or an immunological agent.

In some embodiments of the present disclosure, provided is the pharmaceutical combination, which further comprises one or more pharmaceutically acceptable auxiliary materials; preferably, the pharmaceutical combination further comprises a package insert.

In some embodiments of the present disclosure, provided is the pharmaceutical combination, wherein the molar ratio of the first bispecific protein to the second bispecific protein is (10:1) to (1:10), preferably (5:1) to (1:5), (3:1) to (1:3), or (2:1) to (1:2), and more preferably 1:1.

In some embodiments of the present disclosure, provided is the pharmaceutical combination, wherein the first bispecific protein is the anti-PD-1/anti-VEGFA bispecific antibody VP101(hG1DM) of the present disclosure, the second bispecific protein is the anti-TIGIT antibody-TGF-βR fusion protein TF01T of the present disclosure, and a molar ratio of the first bispecific protein to the second bispecific protein is (10:1) to (1:10), preferably (5:1) to (1:5), (3:1) to (1:3), or (2:1) to (1:2), and more preferably 1:1.

In some embodiments of the present disclosure, the first bispecific protein and the second bispecific protein are active pharmaceutical ingredients (APIs) or active ingredients.

In some embodiments of the present disclosure, provided is the pharmaceutical combination, which consists of the first bispecific protein, the second bispecific protein and a pharmaceutically acceptable auxiliary material.

In some embodiments of the present disclosure, the first bispecific protein, the second bispecific protein and the anti-tumor chemotherapeutic drug are active pharmaceutical ingredients (APIs) or active ingredients.

In some embodiments of the present disclosure, the pharmaceutical combination consists of the first bispecific protein, the second bispecific protein, the anti-tumor chemotherapeutic drug and a pharmaceutically acceptable auxiliary material.

Provided is the pharmaceutical combination according to any one of the aspects of the present disclosure, which is for use in the treatment or prevention of a tumor, wherein
preferably, the tumor is selected from one or more of colon cancer, rectal cancer, lung cancer, liver cancer, ovarian cancer, skin cancer, glioma, melanoma, lymphoma, renal tumor, prostate cancer, bladder cancer, gastrointestinal cancer, breast cancer, brain cancer, cervical cancer, esophageal cancer, urothelial carcinoma, mesothelioma, endometrial cancer, gastric adenocarcinoma, gastroesophageal junction adenocarcinoma, leukemia, multiple myeloma, plasma cell cancer, and pancreatic cancer;
preferably, the lung cancer is non-small cell lung cancer or small cell lung cancer;
preferably, the liver cancer is hepatocellular carcinoma;
preferably, the renal cancer is renal cell carcinoma;
preferably, the breast cancer is triple negative breast cancer;
preferably, the urothelial carcinoma is bladder cancer;
preferably, the lymphoma is non-Hodgkin's lymphoma or B-lymphoma.

Another aspect of the present disclosure relates to a unit formulation, preferably used for treating a tumor, and comprising 1-10,000 mg (preferably 1-1000 mg, preferably 50-500 mg, 100-400 mg, 150-300 mg, 150-250 mg, 200 mg, or 100 mg) of the first bispecific protein according to any one of the aspects of the present disclosure and 1-10,000 mg (preferably 10-1000 mg, preferably 50-500 mg, 100-400 mg, 150-300 mg, 150-250 mg, or 200 mg) of the second bispecific protein according to any one of the aspects of the present disclosure, and optionally one or more of the chemotherapeutic drugs (such as a platinum-based drug and/or a fluorouracil antineoplastic drug) according to the present disclosure, wherein the first bispecific protein, the second bispecific protein and the chemotherapeutic drug are packaged separately.

The present disclosure relates to a method for preventing or treating cancer or a tumor, wherein the method comprises administering to a subject in need one or more unit formulations according to the present disclosure; preferably, the first bispecific protein, the second bispecific protein, and the chemotherapeutic drug in the unit formulation are each administered separately.

Another aspect of the present disclosure relates to a single dose unit, preferably used for treating a tumor, and comprising 0.1-10,000 mg (preferably 1-1000 mg, preferably 50-500 mg, 100-400 mg, 150-300 mg, 150-250 mg, 200 mg, or 100 mg) of the first bispecific protein according to any one of the aspects of the present disclosure and 0.1-10,000 mg (preferably 1-1000 mg, preferably 50-500 mg, 100-400 mg, 150-300 mg, 150-250 mg, 200 mg, or 100 mg) of the second bispecific protein according to any one of the aspects of the present disclosure.

In one or more embodiments of the present disclosure, the first bispecific protein, the second bispecific protein, and/or the chemotherapeutic drug are in a form suitable for intravenous injection or intravenous drip infusion, preferably in a form of a liquid formulation.

In one or more embodiments of the present disclosure, the first bispecific protein according to any one of the aspects of the present disclosure and/or the second bispecific protein according to any one of the aspects of the present disclosure is administered at a single dose of 0.1-100 mg, preferably 1-10 mg per kg body weight; alternatively, the first bispecific protein according to any one of the aspects of the present disclosure and/or the second bispecific protein according to any one of the aspects of the present disclosure is administered at a single dose of 10-1000 mg, preferably 50-500 mg, 100-400 mg, 150-300 mg, 150-250 mg, or 200 mg per subject,
preferably, the administration is performed from twice daily to about once every other day, or once every 3 days, 4 days, 5 days, 6 days, 10 days, 1 week, 2 weeks, 3 weeks, 4 weeks, 5 weeks, or 6 weeks;
preferably, the administration is performed via a route of intravenous drip infusion, intravenous injection, subcutaneous injection, or intraperitoneal injection.

Another aspect of the present disclosure relates to use of the pharmaceutical combination according to any one of the aspects of the present disclosure in the preparation of a medicament for treating or preventing a tumor, wherein
preferably, the tumor is selected from one or more of colon cancer, rectal cancer, lung cancer, liver cancer, ovarian cancer, skin cancer, glioma, melanoma, lymphoma, renal tumor, prostate cancer, bladder cancer, gastrointestinal cancer, breast cancer, brain cancer, cervical cancer, esophageal cancer, urothelial carcinoma, mesothelioma, endometrial cancer, gastric adenocarcinoma, gastroesophageal junction adenocarcinoma, leukemia, multiple myeloma, plasma cell cancer, and pancreatic cancer;
preferably, the lung cancer is non-small cell lung cancer or small cell lung cancer;
preferably, the liver cancer is hepatocellular carcinoma;
preferably, the renal cancer is renal cell carcinoma;
preferably, the breast cancer is triple negative breast cancer;
preferably, the urothelial carcinoma is bladder cancer;
preferably, the lymphoma is non-Hodgkin's lymphoma or B-lymphoma.

Yet another aspect of the present disclosure relates to a method for treating or preventing a tumor, which comprises a step of administering to a subject in need an effective amount of the pharmaceutical combination according to any one of the aspects of the present disclosure, wherein
preferably, the tumor is selected from one or more of colon cancer, rectal cancer, lung cancer, liver cancer, ovarian cancer, skin cancer, glioma, melanoma, lymphoma, renal tumor, prostate cancer, bladder cancer, gastrointestinal cancer, breast cancer, brain cancer, cervical cancer, esophageal cancer, urothelial carcinoma, mesothelioma, endometrial cancer, gastric adenocarcinoma, gastroesophageal junction adenocarcinoma, leukemia, multiple myeloma, plasma cell cancer, and pancreatic cancer;
preferably, the lung cancer is non-small cell lung cancer or small cell lung cancer;
preferably, the liver cancer is hepatocellular carcinoma;
preferably, the renal cancer is renal cell carcinoma;
preferably, the breast cancer is triple negative breast cancer;
preferably, the urothelial carcinoma is bladder cancer;
preferably, the lymphoma is non-Hodgkin's lymphoma or B-lymphoma.

In some embodiments of the present disclosure, provided is the method for treating or preventing a tumor, wherein the first bispecific protein and the second bispecific protein are administered independently before or after surgery and/or before or after radiotherapy.

In some embodiments of the present disclosure, provided is the method for treating or preventing a tumor, wherein
the first bispecific protein and the second bispecific protein are independently administered at a single dose of 0.1-100 mg per kg body weight, preferably 5-50 mg or 5-15 mg per kg body weight;
the first bispecific protein and the second bispecific protein are independently administered once every half day, 1 day, 2 days, 3 days, 4 days, 5 days, 6 days, 10 days, 1 week, 2 weeks, or 3 weeks;
   and/or
the administration is performed via a route of intravenous drip infusion, intravenous injection, subcutaneous injection, or intraperitoneal injection.

Antibody drugs, especially monoclonal antibodies (mAbs), have achieved good efficacy in the treatment of various diseases. Conventional methods for acquiring these therapeutic antibodies include immunizing animals with an antigen and acquiring antibodies targeting the antigen in the immunized animals, or modifying those antibodies with lower affinity for the antigen by affinity maturation.

The variable regions of the light chain and the heavy chain determine the binding of the antigen; the variable region of each chain comprises three hypervariable regions, i.e., complementarity determining regions (CDRs) (the CDRs of the heavy chain (H) include HCDR1, HCDR2, HCDR3, and the CDRs of the light chain (L) include LCDR1, LCDR2, LCDR3; defined by Kabat et al., see Sequences of Proteins of Immunological Interest, Fifth Edition (1991), Volumes 1-3, NIH Publication 91-3242, Bethesda Md).

Preferably, CDRs may also be defined by the IMGT numbering system; see Ehrenmann, Francois, Quentin Kaas, and Marie-Paule Lefranc. "IMGT/3Dstructure-DB and IMGT/DomainGapAlign: a database and a tool for immunoglobulins or antibodies, T cell receptors, MHC, IgSF and MhcSF." Nucleic acids research, 38.suppl_1 (2009): D301-D307.

The amino acid sequences of the CDRs of the monoclonal antibody in (1) to (5) below are analyzed by technical means well known to those skilled in the art, and the results are as follows:
(1) 14C12

The amino acid sequence of the heavy chain variable region is set forth in SEQ ID NO: 20, and the amino acid sequence of the light chain variable region is set forth in SEQ ID NO: 22.

The amino acid sequences of the 3 CDR regions of the heavy chain variable region are as follows:
HCDR1: GFAFSSYD (SEQ ID NO: 1)
HCDR2: ISGGGRYT (SEQ ID NO: 2)
HCDR3: ANRYGEAWFAY (SEQ ID NO: 3)

The amino acid sequences of the 3 CDR regions of the light chain variable region are as follows:
LCDR1: QDINTY (SEQ ID NO: 4)
LCDR2: RAN (SEQ ID NO: 5)
LCDR3: LQYDEFPLT (SEQ ID NO: 6)

### (2) 14C12H1L1

The amino acid sequence of the heavy chain variable region is set forth in SEQ ID NO: 24, and the amino acid sequence of the light chain variable region is set forth in SEQ ID NO: 26.

The amino acid sequences of the 3 CDR regions of the heavy chain variable region are identical to those of 14C12.

The amino acid sequences of the 3 CDR regions of the light chain variable region are identical to those of 14C12.

### (3) 14C12H1L1(M)

The amino acid sequence of the heavy chain variable region is set forth in SEQ ID NO: 24, and the amino acid sequence of the light chain variable region is set forth in SEQ ID NO: 35.

The amino acid sequences of the 3 CDR regions of the heavy chain variable region are identical to those of 14C12.

The amino acid sequences of the 3 CDR regions of the light chain variable region are identical to those of 14C12.

### (4) Bevacizumab

The amino acid sequence of the heavy chain variable region is set forth in SEQ ID NO: 31, and the amino acid sequence of the light chain variable region is set forth in SEQ ID NO: 33.

The amino acid sequences of the 3 CDR regions of the heavy chain variable region are as follows:
HCDR1: GYTFTNYG (SEQ ID NO: 7)
HCDR2: INTYTGEP (SEQ ID NO: 8)
HCDR3: AKYPHYYGSSHWYFDV (SEQ ID NO: 9)

The amino acid sequences of the 3 CDR regions of the heavy chain variable region are as follows:
LCDR1: QDISNY (SEQ ID NO: 10)
LCDR2: FTS (SEQ ID NO: 11)
LCDR3: QQYSTVPWT (SEQ ID NO: 12)

### (5) 26B12

The amino acid sequence of the heavy chain variable region is set forth in SEQ ID NO: 46, and the amino acid sequence of the light chain variable region is set forth in SEQ ID NO: 48.

The amino acid sequences of the 3 CDR regions of the heavy chain variable region are as follows:
HCDR1: GHSFTSDYA (SEQ ID NO: 13)
HCDR2: ISYSDST (SEQ ID NO: 14)
HCDR3: ARLDYGNYGGAMDY (SEQ ID NO: 15)

The amino acid sequences of the 3 CDR regions of the light chain variable region are as follows:
LCDR1: QHVSTA (SEQ ID NO: 16)
LCDR2: SAS (SEQ ID NO: 17)
LCDR3: QQHYITPWT (SEQ ID NO: 18)

The 3 HCDRs and 3 LCDRs of a humanized TIGIT monoclonal antibody are identical to those of the murine TIGIT monoclonal antibody.

In the present disclosure, unless otherwise defined, the scientific and technical terms used herein have the meanings generally understood by those skilled in the art. In addition, the laboratory operations of cell culture, molecular genetics, nucleic acid chemistry, and immunology used herein are the routine procedures widely used in the corresponding fields. Meanwhile, in order to better understand the present disclosure, the definitions and explanations of the related terms are provided below.

As used herein, when referring to the amino acid sequence of PD-1 protein (NCBI GenBank: NP_005009.2), it includes the full length of the PD-1 protein, or the extracellular fragment PD-1ECD of PD-1 or a fragment comprising PD-1ECD, and it also includes a fusion protein of PD-1ECD, such as a fragment fused to an Fc protein fragment of a mouse or human IgG (mFc or hFc). However, those skilled in the art will understand that in the amino acid sequence of the PD-1 protein, mutations or variations (including but not limited to, substitutions, deletions, and/or additions) can be naturally produced or artificially introduced without affecting biological functions thereof. Therefore, in the present disclosure, the term "PD-1 protein" or "PD-1" shall include all such sequences, including natural or artificial variants thereof. In addition, when describing a sequence fragment of the PD-1 protein, it also includes the corresponding sequence fragments in natural or artificial variants thereof.

As used herein, when referring to the amino acid sequence of the VEGFA protein (GenBank ID: NP_001165097.1), it includes the full length of VEGFA protein, and it also includes a fusion protein of VEGFA, such as a fragment fused to an Fc protein fragment of a mouse or human IgG (mFc or hFc). However, those skilled in the art will understand that in the amino acid sequence of the VEGFA protein, mutations or variations (including but not limited to, substitutions, deletions and/or additions) may naturally occur or can be artificially introduced without affecting biological functions thereof. Therefore, in the present disclosure, the term "VEGFA protein" or "VEGFA" shall include all such sequences, including natural or artificial variants thereof. In addition, when describing a sequence fragment of the VEGFA protein, it also includes the corresponding sequence fragments in natural or artificial variants thereof.

As used herein, when referring to the amino acid sequence of the VEGFR2 protein (also known as KDR, GenBank ID: NP_002244), it includes the full length of the VEGFR2 protein, or the extracellular fragment VEGFR2-ECD of VEGFR2 or a fragment comprising VEGFR2-ECD, and it also includes a fusion protein of VEGFR2-ECD, such as a fragment fused to an Fc protein fragment of mouse or human IgG (mFc or hFc). However, those skilled in the art will understand that in the amino acid sequence of the VEGFR2 protein, mutations or variations (including but not limited to, substitutions, deletions and/or additions) may naturally occur or can be artificially introduced without affecting biological functions thereof. Therefore, in the present disclosure, the term "VEGFR2 protein" or "VEGFR2" shall include all such sequences, including natural or artificial variants thereof. In addition, when describing a sequence fragment of the VEGFR2 protein, it also includes the corresponding sequence fragments in natural or artificial variants thereof.

As used herein, unless otherwise specified, the VEGFR is VEGFR1 and/or VEGFR2; specific protein sequence thereof is a sequence known in the prior art, and reference may be made to the sequence disclosed in the existing literature or GenBank. For example, VEGFR1 (VEGFR1, NCBI Gene ID: 2321); VEGFR2 (VEGFR2, NCBI Gene ID: 3791).

As used herein, when referring to the amino acid sequence of TIGIT (NCBI GenBank ID: NP_776160.2), it includes the full length of the TIGIT protein, or an extracellular immunoglobulin variable region (IgV) domain or a fragment comprising an extracellular immunoglobulin variable region (IgV) domain, and it also includes a fusion protein of TIGIT, such as a fragment fused to an Fc protein fragment of mouse or human IgG (mFc or hFc). However, those skilled in the art will understand that in the amino acid sequence of the TIGIT protein, mutations or variations (including but not limited to, substitutions, deletions and/or additions) may naturally occur or can be artificially introduced without affecting biological functions thereof. Therefore, in the present disclosure, the term "TIGIT protein" or "TIGIT" shall include all such sequences, including the sequences set forth and natural or artificial variants thereof. In addition, when describing a sequence fragment of the TIGIT protein, it includes not only the sequence fragment but also the corresponding sequence fragments in natural or artificial variants thereof.

As used herein, the term EC₅₀ refers to the concentration for 50% of maximal effect, i.e., the concentration that can cause 50% of the maximal effect.

As used herein, the term "antibody" refers to an immunoglobulin molecule that generally consists of two pairs of polypeptide chains (each pair consisting of one "light" (L) chain and one "heavy" (H) chain). In a general sense, the heavy chain can be interpreted as a polypeptide chain with a larger molecular weight in an antibody, and the light chain refers to a polypeptide chain with a smaller molecular weight in an antibody. Light chains can be classified into κ and λ light chains. Heavy chains are generally classified into µ, δ, γ, α and ε, and the antibodies are defined as IgM, IgD, IgG, IgA and IgE isotypes, respectively. In light chains and heavy chains, the variable region and constant region are linked via a J region of about 12 or more amino acids, and the heavy chain further comprises a D region of about 3 or more amino acids. Each heavy chain consists of a heavy chain variable region (V_{H}) and a heavy chain constant region (C_{H}). The heavy chain constant region consists of 3 domains (C_{H1}, C_{H2}, and C_{H3}). Each light chain consists of a light chain variable region (V_{L}) and a light chain constant region (C_{L}). The light chain constant region consists of one domain C_{L}. The constant region of the antibody can mediate the binding of immunoglobulins to host tissues or factors, including the binding of various cells of the immune system (e.g., effector cells) to the first component (C1q) of the classical complement system. The V_{H} and V_{L} regions can be further subdivided into hypervariable regions (referred to as complementarity determining regions, or CDRs), and conservative regions referred to as framework regions (FRs) are distributed between the CDRs. Each V_{H} and V_{L} consists of 3 CDRs and 4 FRs arranged from the amino-terminus to the carboxyl-terminus in the following order: FR1, CDR1, FR2, CDR2, FR3, CDR3, FR4. The variable regions (V_{H} and V_{L}) of each heavy chain/light chain pair form an antibody binding site. The assignment of amino acids to each region or domain follows the definition of Chothia & Lesk (1987) J. Mol. Biol. 196: 901-917; and Chothia et al. (1989) Nature 342: 878-883. The term "antibody" is not limited by any specific method for producing the antibody. For example, the antibody includes, in particular, a recombinant antibody, a monoclonal antibody, and a polyclonal antibody. The antibody may be antibodies of different isotypes, such as IgG (e.g., subtype IgG1, IgG2, IgG3, or IgG4), IgA1, IgA2, IgD, IgE, or IgM.

Antigen-binding fragments (e.g., the above-mentioned antibody fragments) of antibodies can be obtained from given antibodies by using conventional techniques known to those skilled in the art (e.g., DNA recombination, or enzymatic or chemical cleavage), and the antigen-binding fragments of the antibodies are screened for specificity in the same way as for intact antibodies.

As used herein, unless otherwise clearly defined in the context, when referring to the term "antibody", it includes not only intact antibodies but also antigen-binding fragments of antibodies.

As used herein, the terms "mAb" and "monoclonal antibody" refer to an antibody or a fragment of an antibody that is derived from a group of highly homologous antibody molecules, i.e., from a group of identical antibody molecules, except for natural mutations that may occur spontaneously. The monoclonal antibody is highly specific for a single epitope on an antigen. The polyclonal antibody, relative to the monoclonal antibody, generally comprises at least two or more different antibodies which generally recognize different epitopes on an antigen. Monoclonal antibodies can generally be obtained using the hybridoma technique first reported by Kohler et al. (Kohler et al., Nature, 256: 495, 1975), but can also be obtained using DNA recombination (see, e.g., U.S. Patent No. 4,816,567).

As used herein, the term "humanized antibody" refers to an antibody or an antibody fragment obtained when all or a part of the CDR regions of a human immunoglobulin (receptor antibody) are replaced by the CDR regions of a non-human antibody (donor antibody), wherein the donor antibody may be a non-human (e.g., mouse, rat, or rabbit) antibody having expected specificity, affinity, or reactivity. In addition, some amino acid residues in the framework regions (FRs) of the receptor antibody can also be replaced by the amino acid residues of corresponding non-human antibodies or by the amino acid residues of other antibodies to further improve or optimize the performance of the antibody. For more details on humanized antibodies, see, for example, Jones et al., Nature, 321: 522-525 (1986); Reichmann et al., Nature, 332: 323-329 (1988); Presta, Curr. Op. Struct. Biol., 2: 593-596 (1992); and Clark, Immunol. Today, 21: 397-402 (2000).

As used herein, the term "epitope" refers to a site on the antigen that an immunoglobulin or antibody specifically binds to. The "epitope" is also referred to in the art as an "antigenic determinant". The epitope or antigenic determinant generally consists of chemically active surface groups of molecules such as amino acids, carbohydrates, or sugar side chains, and usually has specific three-dimensional structural characteristics and specific charge characteristics. For example, an epitope typically comprises at least 3, 4, 5, 6, 7, 8, 9, 10, 11, 12, 13, 14, or 15 contiguous or non-contiguous amino acids in a unique spatial conformation, and it may be "linear" or "conformational". See, e.g., Epitope Mapping Protocols in Methods in Molecular Biology, Vol. 66, G. E. Morris, Ed. (1996). In a linear epitope, all interaction sites between a protein and an interaction molecule (e.g., an antibody) are located linearly along the primary amino acid sequence of the protein. In a conformational epitope, the interaction sites are located across amino acid residues of a protein that are separated from each other.

As used herein, the term "isolated" refers to obtaining by artificial means from a natural state. If a certain "isolated" substance or component is present in nature, it may be the case that a change occurs in its natural environment, or that it is isolated from the natural environment, or both. For example, a certain non-isolated polynucleotide or polypeptide naturally occurs in a certain living animal, and the same polynucleotide or polypeptide with high purity isolated from such a natural state is referred to as an isolated polynucleotide or polypeptide. The term "isolated" does not exclude the existence of artificial or synthetic substances or other impurities that do not affect the activity of the substance.

As used herein, the term *"Escherichia coli* expression system" refers to an expression system consisting of *Escherichia coli* (strain) and a vector, wherein the *Escherichia coli* (strain) is derived from a commercially available strain, such as but not limited to GI698, ER2566, BL21 (DE3), B834 (DE3), and BLR (DE3).

As used herein, the term "vector" refers to a nucleic acid vehicle into which a polynucleotide can be inserted. When a vector allows the expression of the protein encoded by the inserted polynucleotide, the vector is referred to as an expression vector. The vector can be introduced into a host cell by transformation, transduction, or transfection, such that the genetic substance elements carried by the vector can be expressed in the host cell. Vectors are well known to those skilled in the art, including but not limited to: plasmids; phagemids; cosmids; artificial chromosomes, such as yeast artificial chromosome (YAC), bacterial artificial chromosome (BAC), or P1-derived artificial chromosome (PAC); phages such as λ phages or M13 phages; and animal viruses. Animal viruses that can be used as vectors include, but are not limited to, retroviruses (including lentiviruses), adenoviruses, adeno-associated viruses, herpes viruses (such as herpes simplex virus), poxviruses, baculoviruses, papillomaviruses, and papovaviruses (such as SV40). A vector may comprise a variety of elements that control expression, including, but not limited to, promoter sequences, transcription initiation sequences, enhancer sequences, selection elements, and reporter genes. In addition, the vector may further comprise a replication initiation site.

As used herein, the term "host cell" refers to cells to which vectors can be introduced, including, but not limited to, prokaryotic cells such as *Escherichia coli* or *Bacillus subtilis,* fungal cells such as yeast cells or *Aspergillus,* insect cells such as S2 Drosophila cells or Sf9, or animal cells such as fibroblasts, CHO cells, COS cells, NSO cells, HeLa cells, BHK cells, HEK 293 cells or human cells.

As used herein, the term "specifically bind" refers to a non-random binding reaction between two molecules, such as a reaction between an antibody and an antigen it targets. In certain embodiments, an antibody that specifically binds to an antigen (or an antibody that is specific for an antigen) means that the antibody binds to the antigen with an affinity (K_{D}) of less than about 10⁻⁵ M, e.g., less than about 10⁻⁶ M, 10⁻⁷ M, 10⁻⁸ M, 10⁻⁹ M, or 10⁻¹⁰ M or less. In some embodiments of the present disclosure, the term "target" refers to specific binding.

As used herein, the term "K_{D}" refers to a dissociation equilibrium constant for a specific antibody-antigen interaction and is used to describe the binding affinity between the antibody and the antigen. A smaller dissociation equilibrium constant indicates a stronger antibody-antigen binding and a higher affinity between the antibody and the antigen. Generally, an antibody binds to an antigen with a dissociation equilibrium constant (K_{D}) of less than about 10⁻⁵ M, such as less than about 10⁻⁶ M, 10⁻⁷ M, 10-⁸ M, 10⁻⁹ M, or 10⁻¹⁰ M or less, for example, as measured by a BIACORE surface plasmon resonance (SPR) instrument or a Fortebio Octet molecular interaction instrument.

As used herein, the terms "monoclonal antibody" and "mAb" have the same meaning and are used interchangeably; the terms "polyclonal antibody" and "pAb" have the same meaning and are used interchangeably; the terms "polypeptide" and "protein" have the same meaning and are used interchangeably. Besides, in the present disclosure, amino acids are generally represented by single-letter and three-letter abbreviations known in the art. For example, alanine can be represented by A or Ala.

As used herein, the term "pharmaceutically acceptable auxiliary material" or "pharmaceutically acceptable carrier and/or excipient" refers to a carrier and/or excipient that is pharmacologically and/or physiologically compatible with the subject and the active ingredient. Such carriers and/or excipients are well known in the art (see, e.g., Remington's Pharmaceutical Sciences, edited by Gennaro AR, 19th Ed., Pennsylvania, Mack Publishing Company, 1995), including but not limited to: pH regulators, surfactants, adjuvants, and ionic strength enhancers. For example, the pH regulators include, but are not limited to, phosphate buffer; the surfactants include, but are not limited to, cationic, anionic, or non-ionic surfactants, such as Tween-80; the ionic strength enhancers include, but are not limited to, sodium chloride.

As used herein, the term "adjuvant" refers to a non-specific immune enhancer, which can enhance the immune response of an organism to antigens or change the type of immune response when delivered into the organism together with the antigens or in advance. There are various adjuvants, including, but not limited to, aluminum adjuvant (e.g., aluminum hydroxide), Freund's adjuvant (e.g., complete Freund's adjuvant and incomplete Freund's adjuvant), *Corynebacterium parvum,* lipopolysaccharide, cytokine, etc. The Freund's adjuvant is the most commonly used adjuvant in animal experiments. The aluminum hydroxide adjuvant is used more frequently in clinical trials.

As used herein, the term "effective amount" refers to an amount sufficient to obtain or at least partially obtain a desired effect. For example, a prophylactically effective amount refers to an amount sufficient to prevent, stop, or delay the onset of diseases; a therapeutically effective amount refers to an amount sufficient to cure or at least partially stop a disease and complications thereof in patients suffering from the disease. It is undoubtedly within the ability of those skilled in the art to determine such an effective amount. For example, the amount effective for therapeutic purpose will depend on the severity of the disease to be treated, the overall state of the patient's own immune system, the general condition of the patient such as age, body weight, and gender, the route of administration, and other treatments given concurrently, etc.

A "recurrent" cancer is one that regenerates at the original site or a distant site after response to a previous treatment (e.g., surgery). A "locally recurrent" cancer is one that occurs at the same site as the previously treated cancer after treatment.

A "metastatic" cancer refers to one that spreads from one part of the body (e.g., the lungs) to another.

As used herein, the term "completely eliminated" refers to the absence of binding signal or an extremely weak binding signal as detected by an existing instrument (e.g., a Fortebio Octet molecular interaction instrument). In one embodiment of the present disclosure, the absence of binding signal or the extremely weak binding signal refers to a binding signal (i.e., response value or Response) below 0.1 nm.

In the present disclosure, the terms "first" (e.g., first bispecific antibody, first protein functional region, first linker, etc.), "second" (e.g., second bispecific antibody, second protein functional region, second linker, etc.), "third" (e.g., third protein functional region, third linker, etc.), and "fourth" (e.g., fourth protein functional region, fourth linker, etc.) are used for distinguishing or clarity in expression and do not carry typical sequential meanings, unless otherwise specified.

In the present disclosure, "about" or "approximately" means that the value or physical quantity defined fluctuates within a range of 10%, 20%, or 30%, unless otherwise specified. For example, about 100 minutes or approximately 100 minutes may be 90 minutes to 110 minutes, 80 minutes to 120 minutes, or 70 minutes to 130 minutes.

The prior Chinese Patent Application Publication No. CN112830972A is incorporated herein by reference in its entirety.

### Beneficial Effects of the Present Disclosure

The present disclosure achieves one or more of the following technical effects (1) to (2):
(1) The pharmaceutical combination of the present disclosure can effectively prevent or treat a tumor.
(2) The combination of the anti-TIGIT antibody-TGF-βR fusion protein and the anti-PD-1-anti-VEGFA bispecific antibody has a pharmacological effect of effectively inhibiting tumor growth, which is superior to that of the anti-PD-1-anti-VEGFA bispecific antibody or the anti-TIGIT antibody-TGF-βR fusion protein alone, thus achieving a synergistic technical effect.

### BRIEF DESCRIPTION OF THE DRAWINGS

FIG. 1: Promotion of INF-γ secretion in a PBMC + CMV system by a combination of an anti-TIGIT antibody-TGF-βR fusion protein and an anti-PD-1-anti-VEGFA bispecific antibody.
FIG. 2: Promotion of INF-γ secretion in a PBMC + CMV system (+ TGF-β1) by a combination of an anti-TIGIT antibody-TGF-βR fusion protein and an anti-PD-1-anti-VEGFA bispecific antibody.
FIG. 3: Verification of the blocking activity of a combination of an anti-TIGIT antibody-TGF-βR fusion protein and an anti-PD-1-anti-VEGFA bispecific antibody by a reporter gene system.
FIG. 4: Verification of the blocking activity of a combination of an anti-TIGIT antibody-TGF-βR fusion protein and an anti-PD-1-anti-VEGFA bispecific antibody by a reporter gene system (+ VEGF).

Some sequences involved in the present disclosure are as follows:
19. Nucleotide sequence encoding the heavy chain variable region of 14C12: (354 bp) GAGGTCAAACTGGTGGAGAGCGGCGGCGGGCTGGTGAAGCCCGGCGGGTCAC 
TGAAACTGAGCTGCGCCGCTTCCGGCTTCGCCTTTAGCTCCTACGACATGTCA
TGGGTGAGGCAGACCCCTGAGAAGCGCCTGGAATGGGTCGCTACTATCAGCG
GAGGCGGGCGATACACCTACTATCCTGACTCTGTCAAAGGGAGATTCACAATT
AGTCGGGATAACGCCAGAAATACTCTGTATCTGCAGATGTCTAGTCTGCGGTC
CGAGGATACAGCTCTGTACTATTGTGCAAACCGGTACGGCGAAGCATGGTTTG
CCTATTGGGGACAGGGCACCCTGGTGACAGTCTCTGCC (SEQ ID NO: 19)
20. Amino acid sequence of the heavy chain variable region of 14C12: (118 aa) 
EVKLVESGGGLVKPGGSLKLSCAASGFAFSSYDMSWVRQTPEKRLEWVATISGGG
RYTYYPDSVKGRFTISRDNARNTLYLQMSSLRSEDTALYYCANRYGEAWFAYWG
QGTLVTVSA (SEQ ID NO: 20)
21. Nucleotide sequence encoding the light chain variable region of 14C12: (321 bp) 
GACATTAAGATGACACAGTCCCCTTCCTCAATGTACGCTAGCCTGGGCGAGCG
AGTGACCTTCACATGCAAAGCATCCCAGGACATCAACACATACCTGTCTTGGT
TTCAGCAGAAGCCAGGCAAAAGCCCCAAGACCCTGATCTACCGGGCCAATAGA
CTGGTGGACGGGGTCCCCAGCAGATTCTCCGGATCTGGCAGTGGGCAGGATT
ACTCCCTGACCATCAGCTCCCTGGAGTATGAAGACATGGGCATCTACTATTGC
CTGCAGTATGATGAGTTCCCTCTGACCTTTGGAGCAGGCACAAAACTGGAACT
GAAG (SEQ ID NO: 21)
22. Amino acid sequence encoded by the light chain variable region of 14C12: (107 aa) 
DIKMTQSPSSMYASLGERVTFTCKASQDINTYLSWFQQKPGKSPKTLIYRANRLVD
GVPSRFSGSGSGQDYSLTISSLEYEDMGIYYCLQYDEFPLTFGAGTKLELK (SEQ ID
NO: 22)
23. Nucleotide sequence encoding the heavy chain variable region of 14C12H1L1: (354 bp) 
GAAGTGCAGCTGGTCGAGTCTGGGGGAGGGCTGGTGCAGCCCGGCGGGTCAC
TGCGACTGAGCTGCGCAGCTTCCGGATTCGCCTTTAGCTCCTACGACATGTCC
TGGGTGCGACAGGCACCAGGAAAGGGACTGGATTGGGTCGCTACTATCTCAG
GAGGCGGGAGATACACCTACTATCCTGACAGCGTCAAGGGCCGGTTCACAATC
TCTAGAGATAACAGTAAGAACAATCTGTATCTGCAGATGAACAGCCTGAGGGC
TGAGGACACCGCACTGTACTATTGTGCCAACCGCTACGGGGAAGCATGGTTTG
CCTATTGGGGGCAGGGAACCCTGGTGACAGTCTCTAGT (SEQ ID NO: 23)
24. Amino acid sequence of the heavy chain variable region 14C12H1(M)v of 14C12H1L1: (118 aa) 
EVQLVESGGGLVQPGGSLRLSCAASGFAFSSYDMSWVRQAPGKGLDWVATISGG
GRYTYYPDSVKGRFTISRDNSKNNLYLQMNSLRAEDTALYYCANRYGEAWFAYW
GQGTLVTVSS (SEQ ID NO: 24)
25. Nucleotide sequence encoding the light chain variable region of 14C12H1L1: (321 bp) 
GACATTCAGATGACTCAGAGCCCCTCCTCCATGTCCGCCTCTGTGGGCGACAG
GGTCACCTTCACATGCCGCGCTAGTCAGGATATCAACACCTACCTGAGCTGGT
TTCAGCAGAAGCCAGGGAAAAGCCCCAAGACACTGATCTACCGGGCTAATAGA
CTGGTGTCTGGAGTCCCAAGTCGGTTCAGTGGCTCAGGGAGCGGACAGGACT
ACACTCTGACCATCAGCTCCCTGCAGCCTGAGGACATGGCAACCTACTATTGC
CTGCAGTATGATGAGTTCCCACTGACCTTTGGCGCCGGGACAAAACTGGAGCT
GAAG (SEQ ID NO: 25)
26. Amino acid sequence of the light chain variable region of 14C12H1L1: (107 aa) 
DIQMTQSPSSMSASVGDRVTFTCRASQDINTYLSWFQQKPGKSPKTLIYRANRLVS
GVPSRFSGSGSGQDYTLTISSLQPEDMATYYCLQYDEFPLTFGAGTKLELK (SEQ ID
NO: 26)
27. Nucleotide sequence encoding the heavy chain (14C12H1) of 14C12H1L1: (1344 bp) 
GAAGTGCAGCTGGTCGAGTCTGGGGGAGGGCTGGTGCAGCCCGGCGGGTCAC
TGCGACTGAGCTGCGCAGCTTCCGGATTCGCCTTTAGCTCCTACGACATGTCC
TGGGTGCGACAGGCACCAGGAAAGGGACTGGATTGGGTCGCTACTATCTCAG
GAGGCGGGAGATACACCTACTATCCTGACAGCGTCAAGGGCCGGTTCACAATC
TCTAGAGATAACAGTAAGAACAATCTGTATCTGCAGATGAACAGCCTGAGGGC
TGAGGACACCGCACTGTACTATTGTGCCAACCGCTACGGGGAAGCATGGTTTG
CCTATTGGGGGCAGGGAACCCTGGTGACAGTCTCTAGTGCCAGCACCAAAGGA
CCTAGCGTGTTTCCTCTCGCCCCCTCCTCCAAAAGCACCAGCGGAGGAACCGC
TGCTCTCGGATGTCTGGTGAAGGACTACTTCCCTGAACCCGTCACCGTGAGCT
GGAATAGCGGCGCTCTGACAAGCGGAGTCCATACATTCCCTGCTGTGCTGCAA
AGCAGCGGACTCTATTCCCTGTCCAGCGTCGTCACAGTGCCCAGCAGCAGCCT
GGGCACCCAGACCTACATCTGTAACGTCAACCACAAGCCCTCCAACACCAAGG
TGGACAAGAAAGTGGAGCCCAAATCCTGCGACAAGACACACACCTGTCCCCCC
TGTCCTGCTCCCGAACTCCTCGGAGGCCCTAGCGTCTTCCTCTTTCCTCCCAAA
 
CCCAAGGACACCCTCATGATCAGCAGAACCCCTGAAGTCACCTGTGTCGTCGT
GGATGTCAGCCATGAGGACCCCGAGGTGAAATTCAACTGGTATGTCGATGGCG
TCGAGGTGCACAACGCCAAAACCAAGCCCAGGGAGGAACAGTACAACTCCACC
TACAGGGTGGTGTCCGTGCTGACAGTCCTCCACCAGGACTGGCTGAACGGCAA
GGAGTACAAGTGCAAGGTGTCCAACAAGGCTCTCCCTGCCCCCATTGAGAAGA
CCATCAGCAAGGCCAAAGGCCAACCCAGGGAGCCCCAGGTCTATACACTGCCT
CCCTCCAGGGACGAACTCACCAAGAACCAGGTGTCCCTGACCTGCCTGGTCAA
GGGCTTTTATCCCAGCGACATCGCCGTCGAGTGGGAGTCCAACGGACAGCCCG
AGAATAACTACAAGACCACCCCTCCTGTCCTCGACTCCGACGGCTCCTTCTTCC
TGTACAGCAAGCTGACCGTGGACAAAAGCAGGTGGCAGCAGGGAAACGTGTT
CTCCTGCAGCGTGATGCACGAAGCCCTCCACAACCACTACACCCAGAAAAGCC
TGTCCCTGAGCCCCGGCAAA (SEQ ID NO: 27)
28. Amino acid sequence of the heavy chain (14C12H1) of 14C12H1L1: (448 aa) 
EVQLVESGGGLVQPGGSLRLSCAASGFAFSSYDMSWVRQAPGKGLDWVATISGG
GRYTYYPDSVKGRFTISRDNSKNNLYLQMNSLRAEDTALYYCANRYGEAWFAYW
GQGTLVTVSSASTKGPSVFPLAPSSKSTSGGTAALGCLVKDYFPEPVTVSWNSGAL
TSGVHTFPAVLQSSGLYSLSSVVTVPSSSLGTQTYICNVNHKPSNTKVDKKVEPKSC
DKTHTCPPCPAPELLGGPSVFLFPPKPKDTLMISRTPEVTCVVVDVSHEDPEVKFN
WYVDGVEVHNAKTKPREEQYNSTYRVVSVLTVLHQDWLNGKEYKCKVSNKALP
APIEKTISKAKGQPREPQVYTLPPSRDELTKNQVSLTCLVKGFYPSDIAVEWESNG
QPENNYKTTPPVLDSDGSFFLYSKLTVDKSRWQQGNVFSCSVMHEALHNHYTQKS
LSLSPGK (SEQ ID NO: 28)
29. Nucleotide sequence encoding the light chain (14C12L1) of 14C12H1L1: (642 bp) 
GACATTCAGATGACTCAGAGCCCCTCCTCCATGTCCGCCTCTGTGGGCGACAG
GGTCACCTTCACATGCCGCGCTAGTCAGGATATCAACACCTACCTGAGCTGGT
TTCAGCAGAAGCCAGGGAAAAGCCCCAAGACACTGATCTACCGGGCTAATAGA
CTGGTGTCTGGAGTCCCAAGTCGGTTCAGTGGCTCAGGGAGCGGACAGGACT
ACACTCTGACCATCAGCTCCCTGCAGCCTGAGGACATGGCAACCTACTATTGC
CTGCAGTATGATGAGTTCCCACTGACCTTTGGCGCCGGGACAAAACTGGAGCT
GAAGCGAACTGTGGCCGCTCCCTCCGTCTTCATTTTCCCCCCTTCTGACGAACA
GCTGAAATCAGGCACAGCCAGCGTGGTCTGTCTGCTGAACAATTTCTACCCTA
GAGAGGCAAAAGTGCAGTGGAAGGTCGATAACGCCCTGCAGTCCGGCAACAG
CCAGGAGAGTGTGACTGAACAGGACTCAAAAGATAGCACCTATTCCCTGTCTA
 
GTACACTGACTCTGTCCAAGGCTGATTACGAGAAGCACAAAGTGTATGCATGC
GAAGTGACACATCAGGGACTGTCAAGCCCCGTGACTAAGTCTTTTAACCGGGG
CGAATGT (SEQ ID NO: 29)
30. Amino acid sequence of the light chain (14C12L1) of 14C12H1L1: (214 aa) 
DIQMTQSPSSMSASVGDRVTFTCRASQDINTYLSWFQQKPGKSPKTLIYRANRLVS
GVPSRFSGSGSGQDYTLTISSLQPEDMATYYCLQYDEFPLTFGAGTKLELKRTVAA
PSVFIFPPSDEQLKSGTASVVCLLNNFYPREAKVQWKVDNALQSGNSQESVTEQDS
KDSTYSLSSTLTLSKADYEKHKVYACEVTHQGLSSPVTKSFNRGEC (SEQ ID NO: 30)
31. Amino acid sequence of the heavy chain variable region of bevacizumab (bevacizumab-Hv): (123 aa) 


 (SEQ ID NO: 31)
32. Nucleotide sequence encoding the heavy chain variable region of bevacizumab (bevacizumab-Hv): (369 bp) 
GAGGTGCAGCTGGTCGAGTCCGGGGGGGGGCTGGTGCAGCCAGGCGGGTCTC
TGAGGCTGAGTTGCGCCGCTTCAGGGTACACCTTCACAAACTATGGAATGAAT
TGGGTGCGCCAGGCACCAGGAAAGGGACTGGAGTGGGTCGGCTGGATCAACA
CTTACACCGGGGAACCTACCTATGCAGCCGACTTTAAGCGGCGGTTCACCTTC
AGCCTGGATACAAGCAAATCCACTGCCTACCTGCAGATGAACAGCCTGCGAGC
TGAGGACACCGCAGTCTACTATTGTGCTAAATATCCCCACTACTATGGGAGCA
GCCATTGGTATTTTGACGTGTGGGGGCAGGGGACTCTGGTGACAGTGAGCAG
C (SEQ ID NO: 32)
33. Amino acid sequence of the light chain variable region of bevacizumab (bevacizumab-Lv): (107 aa) 

 (SEQ ID
NO: 33)
34. Nucleotide sequence encoding the light chain variable region of bevacizumab (bevacizumab-Lv): (321 bp) 
GATATTCAGATGACTCAGAGCCCCTCCTCCCTGTCCGCCTCTGTGGGCGACAG
GGTCACCATCACATGCAGTGCTTCACAGGATATTTCCAACTACCTGAATTGGTA
TCAGCAGAAGCCAGGAAAAGCACCCAAGGTGCTGATCTACTTCACTAGCTCCC
TGCACTCAGGAGTGCCAAGCCGGTTCAGCGGATCCGGATCTGGAACCGACTTT
ACTCTGACCATTTCTAGTCTGCAGCCTGAGGATTTCGCTACATACTATTGCCAG
CAGTATTCTACCGTGCCATGGACATTTGGCCAGGGGACTAAAGTCGAGATCAA
G (SEQ ID NO: 34)
35. Light chain variable region 14C12L1(M)v of 14C12H1L1(M): (108 aa, with mutation sites underlined in the amino acid sequence based on 14C12H1L1) 
DIQMTQSPSSMSASVGDRVTFTCRASQDINTYLSWFQQKPGKSPKTLIYRANRLVS
GVPSRFSGSGSGQDYTLTISSLQPEDMATYYCLQYDEFPLTFGAGTKLELKR (SEQ
ID NO: 35)
36. Linker
   GGGGSGGGGSGGGGSGGGGS (SEQ ID NO: 36)
37. Linker
   GGGGSGGGGSGGGGS (SEQ ID NO: 37)
38. Amino acid sequence of the heavy chain of the immunoglobulin portion in VP101 (hG1WT): (453 aa) 
EVQLVESGGGLVQPGGSLRLSCAASGYTFTNYGMNWVRQAPGKGLEWVGWINT
YTGEPTYAADFKRRFTFSLDTSKSTAYLQMNSLRAEDTAVYYCAKYPHYYGSSHW
YFDVWGQGTLVTVSSASTKGPSVFPLAPSSKSTSGGTAALGCLVKDYFPEPVTVSW
NSGALTSGVHTFPAVLQSSGLYSLSSVVTVPSSSLGTQTYICNVNHKPSNTKVDKKV
EPKSCDKTHTCPPCPAPELLGGPSVFLFPPKPKDTLMISRTPEVTCVVVDVSHEDPE
VKFNWYVDGVEVHNAKTKPREEQYNSTYRVVSVLTVLHQDWLNGKEYKCKVSN
KALPAPIEKTISKAKGQPREPQVYTLPPSRDELTKNQVSLTCLVKGFYPSDIAVEWE
SNGQPENNYKTTPPVLDSDGSFFLYSKLTVDKSRWQQGNVFSCSVMHEALHNHYT
QKSLSLSPGK (SEQ ID NO: 38)
39. Nucleotide sequence encoding the heavy chain of the immunoglobulin portion in VP101 (hG1WT): (1359 bp) GAGGTGCAGCTGGTCGAGTCCGGGGGGGGGCTGGTGCAGCCAGGCGGGTCTC 
TGAGGCTGAGTTGCGCCGCTTCAGGGTACACCTTCACAAACTATGGAATGAAT
TGGGTGCGCCAGGCACCAGGAAAGGGACTGGAGTGGGTCGGCTGGATCAACA
CTTACACCGGGGAACCTACCTATGCAGCCGACTTTAAGCGGCGGTTCACCTTC
AGCCTGGATACAAGCAAATCCACTGCCTACCTGCAGATGAACAGCCTGCGAGC
TGAGGACACCGCAGTCTACTATTGTGCTAAATATCCCCACTACTATGGGAGCA
GCCATTGGTATTTTGACGTGTGGGGGCAGGGGACTCTGGTGACAGTGAGCAG
CGCAAGCACCAAAGGGCCCAGCGTGTTTCCTCTCGCCCCCTCCTCCAAAAGCA
CCAGCGGAGGAACCGCTGCTCTCGGATGTCTGGTGAAGGACTACTTCCCTGAA
CCCGTCACCGTGAGCTGGAATAGCGGCGCTCTGACAAGCGGAGTCCATACATT
CCCTGCTGTGCTGCAAAGCAGCGGACTCTATTCCCTGTCCAGCGTCGTCACAG
TGCCCAGCAGCAGCCTGGGCACCCAGACCTACATCTGTAACGTCAACCACAAG
CCCTCCAACACCAAGGTGGACAAGAAAGTGGAGCCCAAATCCTGCGACAAGAC
ACACACCTGTCCCCCCTGTCCTGCTCCCGAACTCCTCGGAGGCCCTAGCGTCT
TCCTCTTTCCTCCCAAACCCAAGGACACCCTCATGATCAGCAGAACCCCTGAA
GTCACCTGTGTCGTCGTGGATGTCAGCCATGAGGACCCCGAGGTGAAATTCAA
CTGGTATGTCGATGGCGTCGAGGTGCACAACGCCAAAACCAAGCCCAGGGAG
GAACAGTACAACTCCACCTACAGGGTGGTGTCCGTGCTGACAGTCCTCCACCA
GGACTGGCTGAACGGCAAGGAGTACAAGTGCAAGGTGTCCAACAAGGCTCTC
CCTGCCCCCATTGAGAAGACCATCAGCAAGGCCAAAGGCCAACCCAGGGAGC
CCCAGGTCTATACACTGCCTCCCTCCAGGGACGAACTCACCAAGAACCAGGTG
TCCCTGACCTGCCTGGTCAAGGGCTTTTATCCCAGCGACATCGCCGTCGAGTG
GGAGTCCAACGGACAGCCCGAGAATAACTACAAGACCACCCCTCCTGTCCTCG
ACTCCGACGGCTCCTTCTTCCTGTACAGCAAACTGACCGTCGATAAATCTAGG
TGGCAGCAGGGCAACGTGTTCTCTTGTTCCGTGATGCATGAAGCACTGCACAA
CCATTATACCCAGAAGTCTCTGAGCCTGTCCCCCGGCAAG (SEQ ID NO: 39)
40. Amino acid sequence of the heavy chain of the immunoglobulin portion in VP101 (hG4WT): (450 aa) 
EVQLVESGGGLVQPGGSLRLSCAASGYTFTNYGMNWVRQAPGKGLEWVGWINT
YTGEPTYAADFKRRFTFSLDTSKSTAYLQMNSLRAEDTAVYYCAKYPHYYGSSHW
YFDVWGQGTLVTVSSASTKGPSVFPLAPCSRSTSESTAALGCLVKDYFPEPVTVSW
NSGALTSGVHTFPAVLQSSGLYSLSSVVTVPSSSLGTKTYTCNVDHKPSNTKVDKR
VESKYGPPCPPCPAPEFLGGPSVFLFPPKPKDTLMISRTPEVTCVVVDVSQEDPEVQ
FNWYVDGVEVHNAKTKPREEQFNSTYRVVSVLTVLHQDWLNGKEYKCKVSNKG
LPSSIEKTISKAKGQPREPQVYTLPPSQEEMTKNQVSLTCLVKGFYPSDIAVEWESN
 
GQPENNYKTTPPVLDSDGSFFLYSRLTVDKSRWQEGNVFSCSVMHEALHNHYTQK
SLSLSLGK (SEQ ID NO: 40)
41. Nucleotide sequence encoding the heavy chain of the immunoglobulin portion in VP101(hG4WT): (1350 bp) 
GAGGTGCAGCTGGTCGAGTCCGGGGGGGGGCTGGTGCAGCCAGGCGGGTCTC
TGAGGCTGAGTTGCGCCGCTTCAGGGTACACCTTCACAAACTATGGAATGAAT
TGGGTGCGCCAGGCACCAGGAAAGGGACTGGAGTGGGTCGGCTGGATCAACA
CTTACACCGGGGAACCTACCTATGCAGCCGACTTTAAGCGGCGGTTCACCTTC
AGCCTGGATACAAGCAAATCCACTGCCTACCTGCAGATGAACAGCCTGCGAGC
TGAGGACACCGCAGTCTACTATTGTGCTAAATATCCCCACTACTATGGGAGCA
GCCATTGGTATTTTGACGTGTGGGGGCAGGGGACTCTGGTGACAGTGAGCAG
CGCAAGCACCAAAGGGCCCTCGGTCTTCCCCCTGGCGCCCTGCTCCAGGAGCA
CCTCCGAGAGCACAGCCGCCCTGGGCTGCCTGGTCAAGGACTACTTCCCCGAA
CCGGTGACGGTGTCGTGGAACTCAGGCGCCCTGACCAGCGGCGTGCACACCT
TCCCGGCTGTCCTACAGTCCTCAGGACTCTACTCCCTCAGCAGCGTGGTGACC
GTGCCCTCCAGCAGCTTGGGCACGAAGACCTACACCTGCAACGTAGATCACAA
GCCCAGCAACACCAAGGTGGACAAGAGAGTTGAGTCCAAATATGGTCCCCCAT
GCCCACCATGCCCAGCACCTGAGTTCCTGGGGGGACCATCAGTCTTCCTGTTC
CCCCCAAAACCCAAGGACACTCTCATGATCTCCCGGACCCCTGAGGTCACGTG
CGTGGTGGTGGACGTGAGCCAGGAAGACCCCGAGGTCCAGTTCAACTGGTAC
GTGGATGGCGTGGAGGTGCATAATGCCAAGACAAAGCCGCGGGAGGAGCAGT
TCAACAGCACGTACCGTGTGGTCAGCGTCCTCACCGTCCTGCACCAGGACTGG
CTGAACGGCAAGGAGTACAAGTGCAAGGTCTCCAACAAAGGCCTCCCGTCCTC
CATCGAGAAAACCATCTCCAAAGCCAAAGGGCAGCCCCGAGAGCCACAGGTGT
ACACCCTGCCCCCATCCCAGGAGGAGATGACCAAGAACCAGGTCAGCCTGACC
TGCCTGGTCAAAGGCTTCTACCCCAGCGACATCGCCGTGGAGTGGGAGAGCAA
TGGGCAGCCGGAGAACAACTACAAGACCACGCCTCCCGTGCTGGACTCCGAC
GGCTCCTTCTTCCTCTACAGCAGGCTAACCGTGGACAAGAGCAGGTGGCAGGA
GGGGAATGTCTTCTCATGCTCCGTGATGCATGAGGCTCTGCACAACCACTACA
CACAGAAGTCTCTGAGCCTGTCCCTCGGCAAG (SEQ ID NO: 41)
42. Amino acid sequence of the heavy chain of the immunoglobulin portion in VP101(hG1DM): (453 aa, with mutation sites underlined) EVQLVESGGGLVQPGGSLRLSCAASGYTFTNYGMNWVRQAPGKGLEWVGWINT 
YTGEPTYAADFKRRFTFSLDTSKSTAYLQMNSLRAEDTAVYYCAKYPHYYGSSHW
YFDVWGQGTLVTVSSASTKGPSVFPLAPSSKSTSGGTAALGCLVKDYFPEPVTVSW
NSGALTSGVHTFPAVLQSSGLYSLSSVVTVPSSSLGTQTYICNVNHKPSNTKVDKKV
EPKSCDKTHTCPPCPAPEAAGGPSVFLFPPKPKDTLMISRTPEVTCVVVDVSHEDPE
VKFNWYVDGVEVHNAKTKPREEQYNSTYRVVSVLTVLHQDWLNGKEYKCKVSN
KALPAPIEKTISKAKGQPREPQVYTLPPSRDELTKNQVSLTCLVKGFYPSDIAVEWE
SNGQPENNYKTTPPVLDSDGSFFLYSKLTVDKSRWQQGNVFSCSVMHEALHNHYT
QKSLSLSPGK (SEQ ID NO: 42)
43. Nucleotide sequence encoding the heavy chain of the immunoglobulin portion in VP101(hG1DM): (1359 bp, with mutation sites underlined) 
GAGGTGCAGCTGGTCGAGTCCGGGGGGGGGCTGGTGCAGCCAGGCGGGTCTC
TGAGGCTGAGTTGCGCCGCTTCAGGGTACACCTTCACAAACTATGGAATGAAT
TGGGTGCGCCAGGCACCAGGAAAGGGACTGGAGTGGGTCGGCTGGATCAACA
CTTACACCGGGGAACCTACCTATGCAGCCGACTTTAAGCGGCGGTTCACCTTC
AGCCTGGATACAAGCAAATCCACTGCCTACCTGCAGATGAACAGCCTGCGAGC
TGAGGACACCGCAGTCTACTATTGTGCTAAATATCCCCACTACTATGGGAGCA
GCCATTGGTATTTTGACGTGTGGGGGCAGGGGACTCTGGTGACAGTGAGCAG
CGCAAGCACCAAAGGGCCCAGCGTGTTTCCTCTCGCCCCCTCCTCCAAAAGCA
CCAGCGGAGGAACCGCTGCTCTCGGATGTCTGGTGAAGGACTACTTCCCTGAA
CCCGTCACCGTGAGCTGGAATAGCGGCGCTCTGACAAGCGGAGTCCATACATT
CCCTGCTGTGCTGCAAAGCAGCGGACTCTATTCCCTGTCCAGCGTCGTCACAG
TGCCCAGCAGCAGCCTGGGCACCCAGACCTACATCTGTAACGTCAACCACAAG
CCCTCCAACACCAAGGTGGACAAGAAAGTGGAGCCCAAATCCTGCGACAAGAC
ACACACCTGTCCCCCCTGTCCTGCTCCCGAAGCTGCTGGAGGCCCTAGCGTCT
TCCTCTTTCCTCCCAAACCCAAGGACACCCTCATGATCAGCAGAACCCCTGAA
GTCACCTGTGTCGTCGTGGATGTCAGCCATGAGGACCCCGAGGTGAAATTCAA
CTGGTATGTCGATGGCGTCGAGGTGCACAACGCCAAAACCAAGCCCAGGGAG
GAACAGTACAACTCCACCTACAGGGTGGTGTCCGTGCTGACAGTCCTCCACCA
GGACTGGCTGAACGGCAAGGAGTACAAGTGCAAGGTGTCCAACAAGGCTCTC
CCTGCCCCCATTGAGAAGACCATCAGCAAGGCCAAAGGCCAACCCAGGGAGC
CCCAGGTCTATACACTGCCTCCCTCCAGGGACGAACTCACCAAGAACCAGGTG
TCCCTGACCTGCCTGGTCAAGGGCTTTTATCCCAGCGACATCGCCGTCGAGTG
GGAGTCCAACGGACAGCCCGAGAATAACTACAAGACCACCCCTCCTGTCCTCG
ACTCCGACGGCTCCTTCTTCCTGTACAGCAAACTGACCGTCGATAAATCTAGG
 
TGGCAGCAGGGCAACGTGTTCTCTTGTTCCGTGATGCATGAAGCACTGCACAA
CCATTATACCCAGAAGTCTCTGAGCCTGTCCCCCGGCAAG (SEQ ID NO: 43)
44. Amino acid sequence of the light chain of the immunoglobulin portion in VP101(hG1DM): (214 aa) 
DIQMTQSPSSLSASVGDRVTITCSASQDISNYLNWYQQKPGKAPKVLIYFTSSLHSG
VPSRFSGSGSGTDFTLTISSLQPEDFATYYCQQYSTVPWTFGQGTKVEIKRTVAAPS
VFIFPPSDEQLKSGTASVVCLLNNFYPREAKVQWKVDNALQSGNSQESVTEQDSKD
STYSLSSTLTLSKADYEKHKVYACEVTHQGLSSPVTKSFNRGEC (SEQ ID NO: 44)
45. Nucleotide sequence encoding the light chain of the immunoglobulin portion in VP101(hG1DM): (642 bp) 
GATATTCAGATGACTCAGAGCCCCTCCTCCCTGTCCGCCTCTGTGGGCGACAG
GGTCACCATCACATGCAGTGCTTCACAGGATATTTCCAACTACCTGAATTGGTA
TCAGCAGAAGCCAGGAAAAGCACCCAAGGTGCTGATCTACTTCACTAGCTCCC
TGCACTCAGGAGTGCCAAGCCGGTTCAGCGGATCCGGATCTGGAACCGACTTT
ACTCTGACCATTTCTAGTCTGCAGCCTGAGGATTTCGCTACATACTATTGCCAG
CAGTATTCTACCGTGCCATGGACATTTGGCCAGGGGACTAAAGTCGAGATCAA
GCGGACCGTGGCCGCTCCCAGTGTCTTCATTTTTCCCCCTAGCGACGAACAGC
TGAAATCCGGGACAGCCTCTGTGGTCTGTCTGCTGAACAACTTCTACCCTAGA
GAGGCAAAAGTGCAGTGGAAGGTCGATAACGCCCTGCAGAGTGGCAATTCAC
AGGAGAGCGTGACAGAACAGGACTCCAAAGATTCTACTTATAGTCTGTCAAGC
ACACTGACTCTGAGCAAGGCTGACTACGAAAAGCATAAAGTGTATGCATGTGA
GGTCACCCACCAGGGGCTGAGCAGTCCAGTCACCAAGTCATTCAACAGAGGCG
AGTGC (SEQ ID NO: 45)
46. Amino acid sequence of 26B12VH 
EVQLQESGPGLVKPSQSLSLTCTVTGHSFTSDYAWNWIRQFPGNRLEWMGYISYS
DSTNYNPSLKSRISITRDTSKNQFFLQMNSVTTEDTATYYCARLDYGNYGGAMDY
WGQGTSVTVSS (SEQ ID NO: 46)
47. Nucleotide sequence of 26B12VH 
GAGGTGCAGCTGCAGGAGTCTGGACCTGGCCTGGTGAAACCCTCTCAGTCTCT
GTCCCTCACCTGCACTGTCACTGGCCACTCATTCACCAGTGATTATGCCTGGA
ACTGGATCCGGCAGTTTCCAGGAAACAGACTGGAGTGGATGGGCTACATAAGC
 
TACAGTGATAGCACTAACTACAACCCATCTCTCAAAAGTCGAATCTCTATCACT
CGAGACACATCCAAGAACCAGTTCTTCTTGCAGATGAATTCTGTGACTACTGA
GGACACAGCCACATATTACTGTGCAAGATTGGACTATGGTAACTACGGTGGGG
CTATGGACTACTGGGGTCAAGGGACCTCAGTCACCGTCTCCTCA (SEQ ID NO:
47)
48. Amino acid sequence of 26B12VL 
DIVLTQSHEFMSTSLRDRVSITCKSSQHVSTAVAWYQQKPGQSPKLLIYSASYRYT
GVPDRFTGSGSGTDFTFTISSVKAEDLAVYYCQQHYITPWTFGGGTKLEIK (SEQ ID
NO: 48)
49. Nucleotide sequence of 26B12VL 
GATATTGTGCTAACTCAGTCTCACGAATTCATGTCCACCTCATTACGAGACAGG
GTCAGCATCACCTGCAAATCCAGTCAACATGTGAGTACTGCTGTAGCCTGGTA
TCAACAGAAACCAGGACAATCTCCTAAACTACTGATTTACTCGGCATCCTACCG
GTACACTGGAGTCCCTGATCGCTTCACTGGCAGTGGATCTGGGACGGATTTCA
CTTTCACCATCAGCAGTGTGAAGGCTGAAGACCTGGCAGTTTATTACTGTCAG
CAACATTATATTACTCCGTGGACGTTCGGTGGAGGCACCAAGCTGGAAATAAA
A (SEQ ID NO: 49)
50. Amino acid sequence of 26B12H1VH 
DVQLQESGPGLVKPSQTLSLTCTVSGHSFTSDYAWNWIRQFPGKGLEWIGYISYSD
STNYNPSLKSRITISRDTSKNQFFLQLNSVTAADTATYYCARLDYGNYGGAMDYW
GQGTSVTVSS (SEQ ID NO: 50)
51. Nucleotide sequence of 26B12H1VH 
GATGTGCAGCTGCAGGAGAGCGGCCCCGGACTGGTGAAGCCTTCCCAGACCC
TGTCTCTGACCTGTACAGTGTCTGGCCACAGCTTCACATCCGACTACGCCTGG
AACTGGATCAGGCAGTTTCCAGGCAAGGGCCTGGAGTGGATCGGCTACATCTC
TTATAGCGACTCCACCAACTATAATCCCTCTCTGAAGAGCCGGATCACCATCA
GCAGAGATACATCCAAGAACCAGTTCTTTCTGCAGCTGAACAGCGTGACAGCC
GCCGACACCGCCACATACTATTGCGCCCGGCTGGACTACGGCAATTATGGCGG
AGCCATGGATTACTGGGGCCAGGGCACCTCCGTGACAGTGAGCTCC (SEQ ID
NO: 51)
52. Amino acid sequence of 26B12H2VH 
DVQLQESGPGLVKPSQTLSLTCTVSGHSFTSDYAWSWIRQPPGKGLEWIGYISYSD
STNYNPSLKSRVTISRDTSKNQFSLKLSSVTAADTAVYYCARLDYGNYGGAMDYW
GQGTSVTVSS (SEQ ID NO: 52)
53. Nucleotide sequence of 26B12H2VH 
GATGTGCAGCTGCAGGAGTCTGGCCCAGGACTGGTGAAGCCAAGCCAGACCC
TGTCCCTGACCTGTACAGTGTCCGGCCACTCTTTTACAAGCGACTACGCCTGG
TCTTGGATCAGGCAGCCCCCTGGCAAGGGACTGGAGTGGATCGGCTACATCTC
CTATTCTGACAGCACCAACTATAATCCCTCCCTGAAGTCTCGGGTGACCATCTC
TAGAGATACAAGCAAGAACCAGTTCTCCCTGAAGCTGAGCTCCGTGACCGCAG
CAGACACAGCCGTGTACTATTGCGCCCGGCTGGACTACGGCAATTATGGCGGA
GCCATGGATTACTGGGGCCAGGGCACCAGCGTGACAGTGTCTAGC (SEQ ID NO:
53)
54. Amino acid sequence of 26B12H3VH 
DVQLQESGPGLVKPSQTLSLTCTVSGHSFTSDYAWSWIRQPPGKGLEWIGYISYSD
STNYNPSLKSRVTISVDTSKNQFSLKLSSVTAADTAVYYCARLDYGNYGGAMDYW
GQGTSVTVSS (SEQ ID NO: 54)
55. Nucleotide sequence of 26B12H3VH 
GATGTGCAGCTGCAGGAGTCTGGCCCAGGACTGGTGAAGCCAAGCCAGACCC
TGTCCCTGACCTGTACAGTGTCCGGCCACTCTTTTACAAGCGACTACGCCTGG
TCTTGGATCAGACAGCCCCCTGGCAAGGGACTGGAGTGGATCGGCTACATCTC
CTATTCTGACAGCACCAACTATAATCCCTCCCTGAAGTCTAGAGTGACCATCTC
TGTGGATACAAGCAAGAACCAGTTCTCCCTGAAGCTGAGCTCCGTGACCGCAG
CAGACACAGCCGTGTACTATTGCGCCCGGCTGGACTACGGCAATTATGGCGGA
GCCATGGATTACTGGGGCCAGGGCACCAGCGTGACAGTGTCTAGC (SEQ ID NO:
55)
56. Amino acid sequence of 26B12H4VH 
DVQLQESGPGLVKPSQTLSLTCTVSGHSFTSDYAWNWIRQFPGKGLEWMGYISYS
DSTNYNPSLKSRITISRDTSKNQFFLQLNSVTAADTATYYCARLDYGNYGGAMDY
WGQGTSVTVSS (SEQ ID NO: 56)
**57. Nucleotide sequence of 26B12H4VH** 
GATGTGCAGCTGCAGGAGAGCGGCCCCGGACTGGTGAAGCCTTCCCAGACCC
TGTCTCTGACCTGTACAGTGTCTGGCCACAGCTTCACATCCGACTACGCCTGG
AACTGGATCAGGCAGTTTCCAGGCAAGGGCCTGGAGTGGATGGGCTACATCTC
TTATAGCGACTCCACCAACTATAATCCCTCTCTGAAGAGCCGGATCACCATCA
GCAGAGATACATCCAAGAACCAGTTCTTTCTGCAGCTGAACAGCGTGACAGCC
GCCGACACCGCCACATACTATTGCGCCCGGCTGGACTACGGCAATTATGGCGG
AGCCATGGATTACTGGGGCCAGGGCACCTCCGTGACAGTGAGCTCC (SEQ ID
NO: 57)
**58. Amino acid sequence of 26B12L1VL** 
DIQMTQSPKSLSTSVGDRVTITCRSSQHVSTAVAWYQQKPGKSPKLLIYSASYRYS
GVPDRFSGSGSGTDFTFTISSVQPEDFATYYCQQHYITPWTFGGGTKLEIK (SEQ ID
NO: 58)
**59. Nucleotide sequence of 26B12L1VL** 
GACATCCAGATGACCCAGTCCCCTAAGTCCCTGTCTACAAGCGTGGGCGATCG
GGTGACCATCACATGTAGAAGCTCCCAGCACGTGTCTACCGCAGTGGCATGGT
ACCAGCAGAAGCCAGGCAAGAGCCCTAAGCTGCTGATCTATTCCGCCTCTTAC
AGGTATTCCGGAGTGCCAGACCGGTTTAGCGGCTCCGGCTCTGGCACCGATTT
CACCTTTACAATCTCTAGCGTGCAGCCAGAGGACTTCGCCACATACTATTGCC
AGCAGCACTACATCACCCCATGGACCTTCGGCGGCGGCACAAAGCTGGAGATC
AAG (SEQ ID NO: 59)
**60. Amino acid sequence of 26B12L2VL** 
DIQMTQSPSSLSASVGDRVTITCRSSQHVSTALAWYQQKPGKSPKLLIYSASSRYSG
VPDRFSGSGSGTDFTFTISSLQPEDFATYYCQQHYITPWTFGGGTKLEIK (SEQ ID
NO: 60)
**61. Nucleotide sequence of 26B12L2VL** 
GACATCCAGATGACCCAGTCCCCTAGCTCCCTGTCTGCCAGCGTGGGCGATAG
GGTGACCATCACATGTAGATCTAGCCAGCACGTGTCTACAGCCCTGGCATGGT
ACCAGCAGAAGCCAGGCAAGAGCCCTAAGCTGCTGATCTACTCCGCCTCCTCT
AGGTATTCTGGAGTGCCAGACCGGTTTTCCGGCTCTGGCAGCGGCACCGATTT
CACCTTTACAATCAGCTCCCTGCAGCCAGAGGACTTCGCCACATACTATTGCC
 
AGCAGCACTATATCACCCCATGGACCTTCGGCGGCACCAAGCTGGAGATC
AAG (SEQ ID NO: 61)
**62. Amino acid sequence of 26B12L3VL** 
DIQMTQSPSSLSASVGDRVTITCRASQHVSTALAWYQQKPGKAPKLLIYSASSLQSG
VPSRFSGSGSGTDFTLTISSLQPEDFATYYCQQHYITPWTFGGGTKLEIK (SEQ ID
NO: 62)
**63. Nucleotide sequence of 26B12L3VL** 
GACATCCAGATGACCCAGTCCCCTAGCTCCCTGAGCGCCTCCGTGGGCGATAG
GGTGACCATCACATGTAGAGCCTCTCAGCACGTGAGCACAGCCCTGGCATGGT
ACCAGCAGAAGCCAGGCAAGGCCCCTAAGCTGCTGATCTATAGCGCCTCTAGC
CTGCAGTCCGGAGTGCCATCTCGGTTCTCTGGCAGCGGCTCCGGAACCGACTT
TACCCTGACAATCTCCTCTCTGCAGCCAGAGGATTTCGCCACATACTATTGCCA
GCAGCACTACATCACCCCATGGACCTTCGGCGGCGGCACCAAGCTGGAGATCA
AG (SEQ ID NO: 63)
**64. Amino acid sequence of 26B12L4VL** 
DIQMTQSPKSMSTSVGDRVTITCRSSQHVSTAVAWYQQKPGKSPKLLIYSASYRYS
GVPDRFSGSGSGTDFTFTISSVQPEDFATYYCQQHYITPWTFGGGTKLEIK (SEQ ID
NO: 64)
**65. Nucleotide sequence of 26B12L4VL** 
GACATCCAGATGACCCAGTCCCCTAAGTCCATGTCTACAAGCGTGGGCGACAG
GGTGACCATCACATGTAGAAGCTCCCAGCACGTGTCTACCGCAGTGGCATGGT
ACCAGCAGAAGCCAGGCAAGAGCCCTAAGCTGCTGATCTATTCCGCCTCTTAC
AGGTATTCCGGAGTGCCAGACCGGTTTAGCGGCTCCGGCTCTGGCACCGATTT
CACCTTTACAATCTCTAGCGTGCAGCCAGAGGACTTCGCCACATACTATTGCC
AGCAGCACTACATCACCCCATGGACCTTCGGCGGCGGCACAAAGCTGGAGATC
AAG (SEQ ID NO: 65)
**66. Amino acid sequence of the heavy chain of 26B12H2L2(hG4DM)** 
DVQLQESGPGLVKPSQTLSLTCTVSGHSFTSDYAWSWIRQPPGKGLEWIGYISYSD
STNYNPSLKSRVTISRDTSKNQFSLKLSSVTAADTAVYYCARLDYGNYGGAMDYW
GQGTSVTVSSASTKGPSVFPLAPCSRSTSESTAALGCLVKDYFPEPVTVSWNSGALT
 
SGVHTFPAVLQSSGLYSLSSVVTVPSSSLGTKTYTCNVDHKPSNTKVDKRVESKYG
PPCPPCPAPEAAGGPSVFLFPPKPKDTLMISRTPEVTCVVVDVSQEDPEVQFNWYV
DGVEVHNAKTKPREEQFNSTYRVVSVLTVLHQDWLNGKEYKCKVSNKGLPSSIEK
TISKAKGQPREPQVYTLPPSQEEMTKNQVSLTCLVKGFYPSDIAVEWESNGQPENN
YKTTPPVLDSDGSFFLYSRLTVDKSRWQEGNVFSCSVMHEALHNHYTQKSLSLSL
G (SEQ ID NO: 66)
**67. Nucleotide sequence of the heavy chain of 26B12H2L2(hG4DM)** 
GATGTGCAGCTGCAGGAGTCTGGCCCAGGACTGGTGAAGCCAAGCCAGACCC
TGTCCCTGACCTGTACAGTGTCCGGCCACTCTTTTACAAGCGACTACGCCTGG
TCTTGGATCAGGCAGCCCCCTGGCAAGGGACTGGAGTGGATCGGCTACATCTC
CTATTCTGACAGCACCAACTATAATCCCTCCCTGAAGTCTCGGGTGACCATCTC
TAGAGATACAAGCAAGAACCAGTTCTCCCTGAAGCTGAGCTCCGTGACCGCAG
CAGACACAGCCGTGTACTATTGCGCCCGGCTGGACTACGGCAATTATGGCGGA
GCCATGGATTACTGGGGCCAGGGCACCAGCGTGACAGTGTCTAGCGCCTCCAC
AAAGGGGCCCTCGGTCTTCCCCCTGGCGCCCTGCTCCAGGAGCACCTCCGAGA
GCACAGCCGCCCTGGGCTGCCTGGTCAAGGACTACTTCCCCGAACCGGTGACG
GTGTCGTGGAACTCAGGCGCCCTGACCAGCGGCGTACACACCTTCCCGGCTGT
CCTACAGTCCTCAGGACTCTACTCCCTCAGCAGCGTGGTGACCGTGCCCTCCA
GCAGCTTGGGCACGAAGACCTACACCTGCAACGTAGATCACAAGCCCAGCAAC
ACCAAGGTGGACAAGAGAGTTGAGTCCAAATATGGTCCCCCATGCCCACCATG
CCCAGCACCTGAGGCCGCTGGGGGACCATCAGTCTTCCTGTTCCCCCCAAAAC
CCAAGGACACTCTCATGATCTCCCGGACCCCTGAGGTCACGTGCGTGGTGGTG
GACGTGAGCCAGGAAGACCCCGAGGTCCAGTTCAACTGGTACGTGGATGGCG
TGGAGGTGCATAATGCCAAGACAAAGCCGCGGGAGGAGCAGTTCAACAGCAC
GTACCGTGTGGTCAGCGTCCTCACCGTCCTGCACCAGGACTGGCTGAACGGCA
AGGAGTACAAGTGCAAGGTCTCCAACAAAGGCCTCCCGTCCTCCATCGAGAAA
ACCATCTCCAAAGCCAAAGGGCAGCCCCGAGAGCCACAGGTGTACACCCTGCC
CCCATCCCAGGAGGAGATGACCAAGAACCAGGTCAGCCTGACCTGCCTGGTCA
AAGGCTTCTACCCCAGCGACATCGCCGTGGAGTGGGAGAGCAATGGGCAGCC
GGAGAACAACTACAAGACCACGCCTCCCGTGCTGGACTCCGACGGCTCCTTCT
TCCTCTACAGCAGGCTAACCGTGGACAAGAGCAGGTGGCAGGAGGGGAATGT
CTTCTCATGCTCCGTGATGCATGAGGCTCTGCACAACCACTACACACAGAAGA
GCCTCTCCCTGTCTCTGGGT (SEQ ID NO: 67)
**68. Amino acid sequence of the light chain of 26B12H2L2(hG4DM)** 
DIQMTQSPSSLSASVGDRVTITCRSSQHVSTALAWYQQKPGKSPKLLIYSASSRYSG
VPDRFSGSGSGTDFTFTISSLQPEDFATYYCQQHYITPWTFGGGTKLEIKRTVAAPS
VFIFPPSDEQLKSGTASVVCLLNNFYPREAKVQWKVDNALQSGNSQESVTEQDSKD
STYSLSSTLTLSKADYEKHKVYACEVTHQGLSSPVTKSFNRGEC (SEQ ID NO: 68)
**69. Nucleotide sequence of the light chain of 26B12H2L2(hG4DM)** 
GACATCCAGATGACCCAGTCCCCTAGCTCCCTGTCTGCCAGCGTGGGCGATAG
GGTGACCATCACATGTAGATCTAGCCAGCACGTGTCTACAGCCCTGGCATGGT
ACCAGCAGAAGCCAGGCAAGAGCCCTAAGCTGCTGATCTACTCCGCCTCCTCT
AGGTATTCTGGAGTGCCAGACCGGTTTTCCGGCTCTGGCAGCGGCACCGATTT
CACCTTTACAATCAGCTCCCTGCAGCCAGAGGACTTCGCCACATACTATTGCC
AGCAGCACTATATCACCCCATGGACCTTCGGCGGCACCAAGCTGGAGATC
AAGCGTACGGTGGCAGCCCCATCTGTCTTCATTTTTCCCCCTAGTGACGAGCA
GCTGAAATCCGGAACAGCCTCTGTGGTCTGTCTGCTGAACAATTTCTACCCTC
GCGAAGCCAAGGTGCAGTGGAAAGTCGATAACGCTCTGCAGAGTGGCAATTCA
CAGGAGAGCGTGACTGAACAGGACTCCAAGGATTCTACCTATAGTCTGAGCTC
CACTCTGACCCTGTCCAAAGCAGATTACGAAAAGCACAAAGTGTATGCCTGTG
AGGTCACCCACCAGGGGCTGAGTTCTCCAGTCACCAAATCCTTCAACAGAGGC
GAATGT (SEQ ID NO: 69)
**70. Amino acid sequence of the heavy chain of 26B12H2L2(hG1DM)** 
DVQLQESGPGLVKPSQTLSLTCTVSGHSFTSDYAWSWIRQPPGKGLEWIGYISYSD
STNYNPSLKSRVTISRDTSKNQFSLKLSSVTAADTAVYYCARLDYGNYGGAMDYW
GQGTSVTVSSASTKGPSVFPLAPSSKSTSGGTAALGCLVKDYFPEPVTVSWNSGAL
TSGVHTFPAVLQSSGLYSLSSVVTVPSSSLGTQTYICNVNHKPSNTKVDKKVEPKSC
DKTHTCPPCPAPEAAGGPSVFLFPPKPKDTLMISRTPEVTCVVVDVSHEDPEVKFN
WYVDGVEVHNAKTKPREEQYNSTYRVVSVLTVLHQDWLNGKEYKCKVSNKALP
APIEKTISKAKGQPREPQVYTLPPSRDELTKNQVSLTCLVKGFYPSDIAVEWESNG
QPENNYKTTPPVLDSDGSFFLYSKLTVDKSRWQQGNVFSCSVMHEALHNHYTQKS
LSLSPGK (SEQ ID NO: 70)
**71. Nucleotide sequence of the heavy chain of 26B12H2L2(hG1DM)** 
GATGTGCAGCTGCAGGAGTCTGGCCCAGGACTGGTGAAGCCAAGCCAGACCC
TGTCCCTGACCTGTACAGTGTCCGGCCACTCTTTTACAAGCGACTACGCCTGG
 
TCTTGGATCAGGCAGCCCCCTGGCAAGGGACTGGAGTGGATCGGCTACATCTC
CTATTCTGACAGCACCAACTATAATCCCTCCCTGAAGTCTCGGGTGACCATCTC
TAGAGATACAAGCAAGAACCAGTTCTCCCTGAAGCTGAGCTCCGTGACCGCAG
CAGACACAGCCGTGTACTATTGCGCCCGGCTGGACTACGGCAATTATGGCGGA
GCCATGGATTACTGGGGCCAGGGCACCAGCGTGACAGTGTCTAGCGCCTCTAC
CAAGGGGCCCAGCGTGTTTCCTCTCGCCCCCTCCTCCAAAAGCACCAGCGGAG
GAACCGCTGCTCTCGGATGTCTGGTGAAGGACTACTTCCCTGAACCCGTCACC
GTGAGCTGGAATAGCGGCGCTCTGACAAGCGGAGTCCATACATTCCCTGCTGT
GCTGCAAAGCAGCGGACTCTATTCCCTGTCCAGCGTCGTCACAGTGCCCAGCA
GCAGCCTGGGCACCCAGACCTACATCTGTAACGTCAACCACAAGCCCTCCAAC
ACCAAGGTGGACAAGAAAGTGGAGCCCAAATCCTGCGACAAGACACACACCTG
TCCCCCCTGTCCTGCTCCCGAAGCTGCTGGAGGCCCTAGCGTCTTCCTCTTTC
CTCCCAAACCCAAGGACACCCTCATGATCAGCAGAACCCCTGAAGTCACCTGT
GTCGTCGTGGATGTCAGCCATGAGGACCCCGAGGTGAAATTCAACTGGTATGT
CGATGGCGTCGAGGTGCACAACGCCAAAACCAAGCCCAGGGAGGAACAGTAC
AACTCCACCTACAGGGTGGTGTCCGTGCTGACAGTCCTCCACCAGGACTGGCT
GAACGGCAAGGAGTACAAGTGCAAGGTGTCCAACAAGGCTCTCCCTGCCCCCA
TTGAGAAGACCATCAGCAAGGCCAAAGGCCAACCCAGGGAGCCCCAGGTCTAT
ACACTGCCTCCCTCCAGGGACGAACTCACCAAGAACCAGGTGTCCCTGACCTG
CCTGGTCAAGGGCTTTTATCCCAGCGACATCGCCGTCGAGTGGGAGTCCAACG
GACAGCCCGAGAATAACTACAAGACCACCCCTCCTGTCCTCGACTCCGACGGC
TCCTTCTTCCTGTACAGCAAGCTGACCGTGGACAAAAGCAGGTGGCAGCAGGG
AAACGTGTTCTCCTGCAGCGTGATGCACGAAGCCCTCCACAACCACTACACCC
AGAAAAGCCTGTCCCTGAGCCCCGGCAAA (SEQ ID NO: 71)
**72. Amino acid sequence of an extracellular region fragment (truncated) of TGF-βRII:** 
IPPHVQKSVNNDMIVTDNNGAVKFPQLCKFCDVRFSTCDNQKSCMSNCSITSICEK
PQEVCVAVWRKNDENITLETVCHDPKLPYHDFILEDAASPKCIMKEKKKPGETFF
MCSCSSDECNDNIIFSEEYNTSNPD (SEQ ID NO: 72)
**73. Amino acid sequence of TGF-βRI:** 




 





 (SEQ ID NO: 73)
**74. Amino acid sequence of the extracellular region of TGF-βRI:** 
LQCFCHLCTKDNFTCVTDGLCFVSVTETTDKVIHNSMCIAEIDLIPRDRPFVCAPSS
KTGSVTTTYCCNQDHCNKIELPTTVKSSPGLGPVEL (SEQ ID NO: 74)
**75. Amino acid sequence of TGF-βRII:** 










 (SEQ ID NO: 75)
**76. Amino acid sequence of the extracellular region of TGF-βRII:** 
TIPPHVQKSVNNDMIVTDNNGAVKFPQLCKFCDVRFSTCDNQKSCMSNCSITSICE
KPQEVCVAVWRKNDENITLETVCHDPKLPYHDFILEDAASPKCIMKEKKKPGETF
FMCSCSSDECNDNIIFSEEYNTSNPDLLLVIFQ (SEQ ID NO: 76)
**77. Amino acid sequence of TGF-βRIII:** 
MTSHYVIAIFALMSSCLATAGPEPGALCELSPVSASHPVQALMESFTVLSGCASRGT
TGLPQEVHVLNLRTAGQGPGQLQREVTLHLNPISSVHIHHKSVVFLLNSPHPLVW
HLKTERLATGVSRLFLVSEGSVVQFSSANFSLTAETEERNFPHGNEHLLNWARKEY
GAVTSFTELKIARNIYIKVGEDQVFPPKCNIGKNFLSLNYLAEYLQPKAAEGCVMSS
QPQNEEVHIIELITPNSNPYSAFQVDITIDIRPSQEDLEVVKNLILILKCKKSVNWVIK
 
SFDVKGSLKIIAPNSIGFGKESERSMTMTKSIRDDIPSTQGNLVKWALDNGYSPITSY
TMAPVANRFHLRLENNAEEMGDEEVHTIPPELRILLDPGALPALQNPPIRGGEGQN
GGLPFPFPDISRRVWNEEGEDGLPRPKDPVIPSIQLFPGLREPEEVQGSVDIALSVK
CDNEKMIVAVEKDSFQASGYSGMDVTLLDPTCKAKMNGTHFVLESPLNGCGTRP
RWSALDGVVYYNSIVIQVPALGDSSGWPDGYEDLESGDNGFPGDMDEGDASLFTR
PEIVVFNCSLQQVRNPSSFQEQPHGNITFNMELYNTDLFLVPSQGVFSVPENGHVY
VEVSVTKAEQELGFAIQTCFISPYSNPDRMSHYTIIENICPKDESVKFYSPKRVHFPI
PQADMDKKRFSFVFKPVFNTSLLFLQCELTLCTKMEKHPQKLPKCVPPDEACTSL
DASIIWAMMQNKKTFTKPLAVIHHEAESKEKGPSMKEPNPISPPIFHGLDTLTVMG
IAFAAFVIGALLTGALWYIYSHTGETAGRQQVPTSPPASENSSAAHSIGSTQSTPCSS
SSTA (SEQ ID NO: 77)
**78. Amino acid sequence of the extracellular region of TGF-βRIII:** 
GPEPGALCELSPVSASHPVQALMESFTVLSGCASRGTTGLPQEVHVLNLRTAGQGP
GQLQREVTLHLNPISSVHIHHKSVVFLLNSPHPLVWHLKTERLATGVSRLFLVSEG
SVVQFSSANFSLTAETEERNFPHGNEHLLNWARKEYGAVTSFTELKIARNIYIKVGE
DQVFPPKCNIGKNFLSLNYLAEYLQPKAAEGCVMSSQPQNEEVHIIELITPNSNPYS
AFQVDITIDIRPSQEDLEVVKNLILILKCKKSVNWVIKSFDVKGSLKIIAPNSIGFGK
ESERSMTMTKSIRDDIPSTQGNLVKWALDNGYSPITSYTMAPVANRFHLRLENNAE
EMGDEEVHTIPPELRILLDPGALPALQNPPIRGGEGQNGGLPFPFPDISRRVWNEE
GEDGLPRPKDPVIPSIQLFPGLREPEEVQGSVDIALSVKCDNEKMIVAVEKDSFQAS
GYSGMDVTLLDPTCKAKMNGTHFVLESPLNGCGTRPRWSALDGVVYYNSIVIQVP
ALGDSSGWPDGYEDLESGDNGFPGDMDEGDASLFTRPEIVVFNCSLQQVRNPSSFQ
EQPHGNITFNMELYNTDLFLVPSQGVFSVPENGHVYVEVSVTKAEQELGFAIQTCF
ISPYSNPDRMSHYTIIENICPKDESVKFYSPKRVHFPIPQADMDKKRFSFVFKPVFNT
SLLFLQCELTLCTKMEKHPQKLPKCVPPDEACTSLDASIIWAMMQNKKTFTKPLA
VIHHEAESKEKGPSMKEPNPISPPIFHGLDTLTV (SEQ ID NO: 78)
**79. Amino acid sequence of the heavy chain of tiragolumab:** 
EVQLQQSGPGLVKPSQTLSLTCAISGDSVSSNSAAWNWIRQSPSRGLEWLGKTYYR
FKWYSDYAVSVKGRITINPDTSKNQFSLQLNSVTPEDTAVFYCTRESTTYDLLAGPF
DYWGQGTLVTVSSASTKGPSVFPLAPSSKSTSGGTAALGCLVKDYFPEPVTVSWNS
GALTSGVHTFPAVLQSSGLYSLSSVVTVPSSSLGTQTYICNVNHKPSNTKVDKKVEP
KSCDKTHTCPPCPAPELLGGPSVFLFPPKPKDTLMISRTPEVTCVVVDVSHEDPEV
KFNWYVDGVEVHNAKTKPREEQYNSTYRVVSVLTVLHQDWLNGKEYKCKVSNK
 
ALPAPIEKTISKAKGQPREPQVYTLPPSREEMTKNQVSLTCLVKGFYPSDIAVEWES
NGQPENNYKTTPPVLDSDGSFFLYSKLTVDKSRWQQGNVFSCSVMHEALHNHYTQ
KSLSLSPGK (SEQ ID NO: 79)
**80. Amino acid sequence of the light chain of tiragolumab:** 
DIVMTQSPDSLAVSLGERATINCKSSQTVLYSSNNKKYLAWYQQKPGQPPNLLIYW
ASTRESGVPDRFSGSGSGTDFTLTISSLQAEDVAVYYCQQYYSTPFTFGPGTKVEIK
RTVAAPSVFIFPPSDEQLKSGTASVVCLLNNFYPREAKVQWKVDNALQSGNSQESV
TEQDSKDSTYSLSSTLTLSKADYEKHKVYACEVTHQGLSSPVTKSFNRGEC (SEQ
ID NO: 80)
**81. Amino acid sequence of the heavy chain variable region of RG6058(hG4):** 
EVQLQQSGPGLVKPSQTLSLTCAISGDSVSSNSAAWNWIRQSPSRGLEWLGKTYYR
FKWYSDYAVSVKGRITINPDTSKNQFSLQLNSVTPEDTAVFYCTRESTTYDLLAGPF
DYWGQGTLVTVSS (SEQ ID NO: 81)
**82. Amino acid sequence of the light chain variable region of RG6058(hG4):** 
DIVMTQSPDSLAVSLGERATINCKSSQTVLYSSNNKKYLAWYQQKPGQPPNLLIYW
ASTRESGVPDRFSGSGSGTDFTLTISSLQAEDVAVYYCQQYYSTPFTFGPGTKVEIK
(SEQ ID NO: 82)
**83. Amino acid sequence of the linker:**
   **GGGGSGGGGSGGGGSGGGGSG (SEQ ID NO: 83)**
**84. Amino acid sequence of the heavy chain of an anti-HEL &TGFβ antibody** 
EVQLEQSGAELMKPGASVKISCKATGYTFTTYWIEWIKQRPGHSLEWIGEILPGSD
STYYNEKVKGKVTFTADASSNTAYMQLSSLTSEDSAVYYCARGDGFYVVYWGQGT
TLTVSSASTKGPSVFPLAPCSRSTSESTAALGCLVKDYFPEPVTVSWNSGALTSGVH
TFPAVLQSSGLYSLSSVVTVPSSSLGTKTYTCNVDHKPSNTKVDKRVESKYGPPCPP
CPAPEAAGGPSVFLFPPKPKDTLMISRTPEVTCVVVDVSQEDPEVQFNWYVDGVEV
HNAKTKPREEQFNSTYRVVSVLTVLHQDWLNGKEYKCKVSNKGLPSSIEKTISKA
KGQPREPQVYTLPPSQEEMTKNQVSLTCLVKGFYPSDIAVEWESNGQPENNYKTT
PPVLDSDGSFFLYSRLTVDKSRWQEGNVFSCSVMHEALHNHYTQKSLSLSLGGGG
GSGGGGSGGGGSGGGGSGIPPHVQKSVNNDMIVTDNNGAVKFPQLCKFCDVRFST
CDNQKSCMSNCSITSICEKPQEVCVAVWRKNDENITLETVCHDPKLPYHDFILEDA
 ASPKCIMKEKKKPGETFFMCSCSSDECNDNIIFSEEYNTSNPD (SEQ ID NO: 84)
**85. Amino acid sequence of the light chain of an anti-HEL & TGFβ antibody** 
DIELTQSPATLSVTPGDSVSLSCRASQSISNNLHWYQQKSHESPRLLIKYTSQSMSGI
PSRFSGSGSGTDFTLSINSVETEDFGVYFCQQSGSWPRTFGGGTKLDIKRTVAAPSV
FIFPPSDEQLKSGTASVVCLLNNFYPREAKVQWKVDNALQSGNSQESVTEQDSKDS
TYSLSSTLTLSKADYEKHKVYACEVTHQGLSSPVTKSFNRGEC (SEQ ID NO: 85)
**86. Amino acid sequence of the heavy chain of RG6058(G1DM)** 
EVQLQQSGPGLVKPSQTLSLTCAISGDSVSSNSAAWNWIRQSPSRGLEWLGKTYYR
FKWYSDYAVSVKGRITINPDTSKNQFSLQLNSVTPEDTAVFYCTRESTTYDLLAGPF
DYWGQGTLVTVSSASTKGPSVFPLAPSSKSTSGGTAALGCLVKDYFPEPVTVSWNS
GALTSGVHTFPAVLQSSGLYSLSSVVTVPSSSLGTQTYICNVNHKPSNTKVDKKVEP
KSCDKTHTCPPCPAPEAAGGPSVFLFPPKPKDTLMISRTPEVTCVVVDVSHEDPEV
KFNWYVDGVEVHNAKTKPREEQYNSTYRVVSVLTVLHQDWLNGKEYKCKVSNK
ALPAPIEKTISKAKGQPREPQVYTLPPSRDELTKNQVSLTCLVKGFYPSDIAVEWES
NGQPENNYKTTPPVLDSDGSFFLYSKLTVDKSRWQQGNVFSCSVMHEALHNHYTQ
KSLSLSPGK (SEQ ID NO: 86)
**87. Amino acid sequence of the light chain of RG6058(G1DM)** 
DIVMTQSPDSLAVSLGERATINCKSSQTVLYSSNNKKYLAWYQQKPGQPPNLLIYW
ASTRESGVPDRFSGSGSGTDFTLTISSLQAEDVAVYYCQQYYSTPFTFGPGTKVEIK
RTVAAPSVFIFPPSDEQLKSGTASVVCLLNNFYPREAKVQWKVDNALQSGNSQESV
TEQDSKDSTYSLSSTLTLSKADYEKHKVYACEVTHQGLSSPVTKSFNRGEC (SEQ
ID NO: 87)
**88. Amino acid sequence of a TGF-βRII extracellular region fragment variant of TF01T:** 
IPPHVQKTVNNDMIVTDNNGAVKFPQLCKFCDVRFSTCDNQKSCMSNCSITSICEK
PQEVCVAVWRKNDENITLETVCHDPKLPYHDFILEDAASPKCIMKEKKKPGETFF
MCSCSSDECNDNIIFSEEYNTSNPD (SEQ ID NO: 88)
**89. Amino acid sequence of the heavy chain constant region of hIgG4WT** 
ASTKGPSVFPLAPCSRSTSESTAALGCLVKDYFPEPVTVSWNSGALTSGVHTFPAVL
QSSGLYSLSSVVTVPSSSLGTKTYTCNVDHKPSNTKVDKRVESKYGPPCPPCPAPEF
LGGPSVFLFPPKPKDTLMISRTPEVTCVVVDVSQEDPEVQFNWYVDGVEVHNAKT
 
KPREEQFNSTYRVVSVLTVLHQDWLNGKEYKCKVSNKGLPSSIEKTISKAKGQPR
EPQVYTLPPSQEEMTKNQVSLTCLVKGFYPSDIAVEWESNGQPENNYKTTPPVLDS
DGSFFLYSRLTVDKSRWQEGNVFSCSVMHEALHNHYTQKSLSLSLG (SEQ ID NO:
89)
**90. Amino acid sequence of the heavy chain constant region of hIgG1WT** 





 (SEQ
ID NO: 90)
**91. A repeating unit in a linker**
   **GGGGS (SEQ ID NO: 91)**
**92. A fragment in a linker**
   **GGGGSG (SEQ ID NO: 92)**

### DETAILED DESCRIPTION

Embodiments of the present disclosure will be described in detail below with reference to examples, but those skilled in the art will understand that the following examples are only for illustrating the present disclosure and should not be construed as limitations to the scope of the present disclosure. The experimental procedures without specified conditions in the examples are conducted under conventional conditions or conditions recommended by the manufacturer. The used reagents or instruments without specified manufacturers are all commercially available conventional products.

The sequence of the isotype control antibody, human anti-hen egg lysozyme IgG (anti-HEL antibody, or human IgG, abbreviated as hIgG) is derived from the variable region sequence of the Fab F10.6.6 sequence in the study reported by Acierno et al., entitled "Affinity maturation increases the stability and plasticity of the Fv domain of anti-protein antibodies" (Acierno et al., J Mol Biol., 2007; 374(1): 130-146). The hIgG1DM used in the examples is an isotype control antibody of anti-HEL with an hG1DM constant region sequence, prepared in Akeso Biopharma Inc., Batch No: 20190520.

Jurkat-NFAT-PD1-hTIGIT is a cell expressing human TIGIT constructed by Akeso Biopharma Inc. on the basis of Jurkat-NFAT-PD1 cells (PD1 Effector cells, Promega, Cat. No. J112A) by transfection.

### Preparation Example 1: Sequence Design of Anti-PD-1 Antibody 14C12 and Humanized Antibody 14C12H1L1 Thereof

The amino acid sequences and encoding nucleotide sequences of the heavy and light chains of the anti-PD-1 antibody 14C12 and the humanized antibody 14C12H1L1 thereof are identical to those of 14C12 and 14C12H1L1 in Chinese Patent Publication No. CN112830972A, respectively.
(1) Heavy and light chain variable region sequences of 14C12
   Nucleotide sequence encoding the heavy chain variable region of 14C12: SEQ ID NO: 19;
   amino acid sequence of the heavy chain variable region of 14C12: SEQ ID NO: 20;
   nucleotide sequence encoding the light chain variable region of 14C12: SEQ ID NO: 21; and
   amino acid sequence encoded by the light chain variable region of 14C12: SEQ ID NO: 22.
(2) Heavy and light chain variable region sequences and heavy and light chain sequences of humanized monoclonal antibody 14C12H1L1
   Nucleotide sequence encoding the heavy chain variable region of 14C12H1L1: SEQ ID NO: 23;
   amino acid sequence of the heavy chain variable region of 14C12H1L1: SEQ ID NO: 24;
   nucleotide sequence encoding the light chain variable region of 14C12H1L1: SEQ ID NO: 25;
   amino acid sequence of the light chain variable region of 14C12H1L1: SEQ ID NO: 26;
   nucleotide sequence encoding the heavy chain (14C12H1) of 14C12H1L1: SEQ ID NO: 27;
   amino acid sequence of the heavy chain (14C12H1) of 14C12H1L1: SEQ ID NO: 28;
   nucleotide sequence encoding the light chain (14C12L1) of 14C12H1L1: SEQ ID NO: 29; and
   amino acid sequence of the light chain (14C12L1) of 14C12H1L1: SEQ ID NO: 30.

### Preparation Example 2: Preparation of Anti-VEGFA Antibody Bevacizumab

Reference was made to the Chinese Patent Publication No. CN1259962A for amino acid sequences of the heavy chain variable region and the light chain variable region of the commercially available anti-VEGFA monoclonal antibody Avastin (bevacizumab). Genscript was entrusted to synthesize nucleotide sequences encoding the heavy chain variable region and the light chain variable region.

Amino acid sequence of the heavy chain variable region (bevacizumab-Hv) of bevacizumab: SEQ ID NO: 31;
nucleotide sequence encoding the heavy chain variable region (Bevacizumab-Hv) of bevacizumab: SEQ ID NO: 32;
amino acid sequence of the light chain variable region (bevacizumab-Lv) of bevacizumab: SEQ ID NO: 33; and
nucleotide sequence encoding the light chain variable region (bevacizumab-Lv) of bevacizumab: SEQ ID NO: 34.

The heavy chain constant regions were all Ig gamma-1 chain C region (ACCESSION: P01857); the light chain constant regions were all Ig kappa chain C region (ACCESSION: P01834).

The heavy chain cDNA and the light chain cDNA of bevacizumab were cloned into the vector pcDNA3.1, and the recombinant expression plasmid of the antibody bevacizumab was obtained. The recombinant plasmid was transfected into 293F cells. The 293F cell culture was purified and then detected.

The anti-VEGFA monoclonal antibody Avastin (bevacizumab) was thus obtained.

### Preparation Example 3: Sequence Design of Mutant 14C12H1L1(M) of Anti-PD-1 Humanized Antibody 14C12H1L1

On the basis of 14C12H1L1, individual amino acids in the framework region (light chain) thereof were mutated to obtain 14C12H1L1(M). Heavy chain variable region 14C12H1L1(M)ᵥ of 14C12H1(M): identical to the heavy chain variable region 14C12H1 of 14C12H1L1, i.e., the amino acid sequence is set forth in SEQ ID NO: 24.

Light chain variable region of 14C12H1L1(M)ᵥ of 14C12L1(M): SEQ ID NO: 35.

### Preparation Example 4: Sequence Design of Bispecific Antibodies

### 1. Sequence design

The structure of the bispecific antibodies described herein is in the Morrison format (IgG-scFv), i.e., C-termini of two heavy chains of an IgG antibody are each linked to a scFv fragment of another antibody. The main composition design of the heavy and light chains is shown in Table 1 below.

On the basis of the bevacizumab described above, the VP101 antibody, comprising the amino acid sequences of the heavy chain variable region and the light chain variable region of the 14C12H1L1(M) described above as the ScFv fragments, is referred to as VP101(M). Compared with 14C12H1L1, 14C12H1L1(M) demonstrated effectively optimized bispecific antibody structure and improved efficacy.

**Table 1: Composition design of heavy and light chains of VP101(M) and VP101(G4M)**

| **Bispecific antibody No.** | **Immunoglobulin portion** | | **Linker** | **scFv portion** |
|---|---|---|---|---|
| | **Heavy chain** | **Light chain** | | |
| **VP101(M)** | **Bevacizumab-H** | **Bevacizumab-L** | **Linker2** | **14C12H1(M)v-Linker2-14C12L1(M)v** |
| **VP101(G4M)** | **Bevacizumab-G4H** | **Bevacizumab-L** | **Linker2** | **14C12H1(M)v- Linker2-14C12L1(M)v** |

In Table 1 above:
(1) Those with a "V" mark in the bottom right corner refer to the variable regions of the corresponding heavy chains or the variable regions of the corresponding light chains. For those without a "V" mark, the corresponding heavy or light chain is the full length comprising the constant region. Reference was made to the corresponding sequences described in the above preparation examples for the amino acid sequences and encoding nucleotide sequences thereof of these variable regions or full-length sequences.
(2) The amino acid sequence of Linker2 is SEQ ID NO: 36.
   Optionally, SEQ ID NO: 37 may be used as Linker1, replacing the aforementioned Linker 2.
(3) Bevacizumab-H comprises Ig gamma-1 chain C region (ACCESSION: P01857) as the heavy chain constant region.
(4) Bevacizumab-G4H comprises Ig gamma-4 chain C region (ACCESSION: P01861.1) as the heavy chain constant region.

### 2. Expression and purification of antibody VP101(M)

The heavy chain cDNA sequence and the light chain cDNA sequence of VP101(M) were each cloned into the pUC57simple vector (provided by Genscript) to obtain plasmids pUC57simple-VP101H and pUC57simple-VP101L, respectively.

The plasmids pUC57simple-VP101H and pUC57simple-VP101L were each subjected to enzyme cleavage (HindIII & EcoRI). The heavy and light chains were recovered by electrophoresis, and then each subcloned into the pcDNA3.1 vector. The recombinant plasmids were extracted and co-transfected into 293F cells. After 7 days of cell culture, the culture medium was subjected to high-speed centrifugation, and the supernatant was concentrated and then loaded onto a HiTrap MabSelect SuRe column. The protein was eluted in one step with an elution buffer. The target sample antibody VP101 was recovered and buffer-exchanged into PBS.

### 3. Detection of antibody VP101(M)

The purified sample was added to both a reduced protein electrophoresis loading buffer and a non-reduced protein electrophoresis loading buffer, boiled and subjected to SDS-PAGE electrophoresis analysis.

For differentiation from the mutant antibody in Preparation Example 5 below, VP101(M) is also referred to as VP101(hG1WT) in the present disclosure. VP101(M) described above is the "wild-type", comprising an Ig gamma-1 chain C region (ACCESSION: P01857) as the heavy chain constant region and an Ig kappa chain C region (ACCESSION: P01834) as the light chain constant region.

Amino acid sequence of the heavy chain (bevacizumab-H) of the immunoglobulin portion in VP101(hG1WT): SEQ ID NO: 38; and nucleotide sequence encoding the heavy chain of the immunoglobulin portion in VP101(hG1WT): SEQ ID NO: 39.

For differentiation from the mutant antibody in Preparation Example 5 below, VP101(G4M) is also referred to as VP101(hG4WT) in the present disclosure. VP101(G4M) described above is the "wild-type", comprising an Ig gamma-4 chain C region (ACCESSION: P01861.1) as the heavy chain constant region and an Ig kappa chain C region (ACCESSION: P01834) as the light chain constant region.

Amino acid sequence of the heavy chain of the immunoglobulin portion in VP101 (hG4WT): SEQ ID NO: 40; and
nucleotide sequence encoding the heavy chain of the immunoglobulin portion in VP101(hG4WT): SEQ ID NO: 41.

### Preparation Example 5: Non-Variable Region Amino Acid Mutation Design Based on Humanized Bispecific Antibody VP101(hG1WT)

On the basis of VP101(hG1WT) obtained in Preparation Example 4, the inventors introduced a leucine-to-alanine point mutation at position 234 (L234A) and a leucine-to-alanine point mutation at position 235 (L235A) of the heavy chain thereof to obtain VP101 (hG1DM).

Amino acid sequence (453 aa, with mutation sites underlined) of the heavy chain of the immunoglobulin portion in VP101(hG1DM): SEQ ID NO: 42; and
nucleotide sequence (1359 bp, with mutation sites underlined) encoding the heavy chain of the immunoglobulin portion in VP101(hG1DM): SEQ ID NO: 43.

The amino acid sequences of the light chains of the immunoglobulin portions of VP101(hG1DM), VP101(hG1WT), and VP101(hG4WT) are identical, and the encoding nucleotide sequences thereof are also identical. Amino acid sequence of the light chain of the immunoglobulin portion in VP101(hG1DM): SEQ ID NO: 44; and
nucleotide sequence encoding the light chain of the immunoglobulin portion in VP101(hG1DM): SEQ ID NO: 45.

### Preparation Example 6: Preparation of Anti-TIGIT Antibodies

### 1. Preparation of hybridoma cell line LT019

The antigen used for preparing the anti-TIGIT antibody was human TIGIT-mFc (TIGIT was Genbank ID: NP_776160.2). Spleen cells of immunized mice were fused with mouse myeloma cells to prepare hybridoma cells. With human TIGIT-hFc as an antigen, the hybridoma cells were screened by indirect ELISA to obtain hybridoma cells capable of secreting an antibody that specifically binds to TIGIT. The hybridoma cells obtained by screening were subjected to a limiting dilution assay to obtain a stable hybridoma cell line. The above hybridoma cell line was named hybridoma cell line LT019, and the monoclonal antibody secreted by the cell line was named 26B12.

Hybridoma cell line LT019 (also referred to as TIGIT-26B12) was deposited at the China Center for Type Culture Collection (CCTCC) on October 23, 2020. The CCTCC designation was CCTCC NO. C2020208, and the deposition address was Wuhan University, Wuhan, China, postal code: 430072.

### 2. Preparation of anti-TIGIT antibody 26B12

The LT019 cell line prepared above was cultured in a chemical defined medium (CD medium, containing 1% penicillin-streptomycin) at 37 °C with 5% CO2. After 7 days, the cell culture supernatant was collected, subjected to high-speed centrifugation and vacuum filtration through a microfiltration membrane, and purified using a HiTrap protein A HP column to obtain antibody 26B12.

### Preparation Example 7: Sequence Analysis of Anti-TIGIT Antibody 26B12

mRNA was extracted from the cell line LT019 cultured in Preparation Example 6 according to the method described in the manual of RNAprep pure Cell/Bacteria Kit (Tiangen, Cat. No. DP430).

cDNA was synthesized according to the instructions of an Invitrogen SuperScript^{®} III First-Strand Synthesis System for RT-PCR kit and amplified by PCR.

The PCR amplification product was directly subjected to TA cloning according to the instructions of a pEASY-T1 Cloning Kit (Transgen CT101).

The TA cloning products were directly sequenced, and the sequencing results are as follows:
The nucleotide sequence of the heavy chain variable region is set forth in SEQ ID NO: 47, with a fragment length of 363 bp.

The amino acid sequence encoded thereby is set forth in SEQ ID NO: 46, with a length of 121 amino acids.

The sequence of heavy chain HCDR1 is set forth in SEQ ID NO: 13, the sequence of HCDR2 is set forth in SEQ ID NO: 14, and the sequence of HCDR3 is set forth in SEQ ID NO: 15.

The nucleotide sequence of the light chain variable region is set forth in SEQ ID NO: 49, with a length of 321 bp.

The amino acid sequence encoded thereby is set forth in SEQ ID NO: 48, with a length of 107 amino acids.

The sequence of light chain LCDR1 is set forth in SEQ ID NO: 16, the sequence of LCDR2 is set forth in SEQ ID NO: 17, and the sequence of LCDR3 is set forth in SEQ ID NO: 18.

### Preparation Example 8: Design and Preparation of Anti-Human TIGIT Humanized Antibodies and Mutant Antibodies

1. Design of light and heavy chains of anti-human TIGIT humanized antibodies 26B12H1L1, 26B12H4L1, 26B12H2L2, 26B12H3L2, 26B12H2L3, 26B12H3L3, 26B12H1L4, and 26B12H4L4

Based on the three-dimensional crystal structure of human TIGIT protein and the sequences of antibody 26B12 obtained in Preparation Example 7, the variable region sequences of antibodies 26B12H1L1, 26B12H4L1, 26B12H2L2, 26B12H3L2, 26B12H2L3, 26B12H3L3, 26B12H1L4, and 26B12H4L4 (for the antibody constant region sequences, the heavy chain constant region was the Ig gamma-1 chain C region; the light chain constant region was the Ig kappa chain C region) were designed.

The designed variable region sequences are shown in Table 2 below.

**Table 2: Variable region sequences of anti-human TIGIT humanized antibodies**

| **Name** | **Amino acid sequence of heavy chain variable region SEQ ID NO:** | **Nucleotide sequence of heavy chain variable region SEQ ID NO:** | **Amino acid sequence of light chain variable region SEQ ID NO:** | **Nucleotide sequence of light chain variable region SEQ ID NO:** |
|---|---|---|---|---|
| **26B12H1L1** | **50** | **51** | **58** | **59** |
| **26B12H4L1** | **56** | **57** | **58** | **59** |
| **26B12H2L2** | **52** | **53** | **60** | **61** |
| **26B12H2L3** | **52** | **53** | **62** | **63** |
| **26B12H3L2** | **54** | **55** | **60** | **61** |
| **26B12H3L3** | **54** | **55** | **62** | **63** |
| **26B12H1L4** | **50** | **51** | **64** | **65** |
| **26B12H4L4** | **56** | **57** | **64** | **65** |

For the above 8 antibodies 26B12H1L1, 26B12H4L1, 26B12H2L2, 26B12H3L2, 26B12H2L3, 26B12H3L3, 26B12H1L4, and 26B12H4L4, the nucleotide sequences of the heavy chain variable regions are all 363 bp in length, and the amino acid sequences encoded thereby are all 121 aa in length; the nucleotide sequences of the light chain variable regions are all 321 bp in length, and the amino acid sequences encoded thereby are all 107 aa in length.

Moreover, the above 8 antibodies have the same HCDR1-HCDR3 and LCDR1-LCDR3 as follows:
The sequence of HCDR1 is set forth in SEQ ID NO: 13, the sequence of HCDR2 is set forth in SEQ ID NO: 14, and the sequence of HCDR3 is set forth in SEQ ID NO: 15;
the sequence of LCDR1 is set forth in SEQ ID NO: 16, the sequence of LCDR2 is set forth in SEQ ID NO: 17, and the sequence of LCDR3 is set forth in SEQ ID NO: 18.

2. Preparation of humanized antibodies 26B12H1L1, 26B12H4L1, 26B12H2L2, 26B12H3L2, 26B12H2L3, 26B12H3L3, 26B12H1L4, and 26B12H4L4

The heavy chain constant regions were all the Ig gamma-1 chain C region (ACCESSION: P01857), and the light chain constant regions were all the Ig kappa chain C region (ACCESSION: P01834).

The heavy chain cDNA and light chain cDNA of 26B12H1L1, the heavy chain cDNA and light chain cDNA of 26B12H4L1, the heavy chain cDNA and light chain cDNA of 26B12H2L2, the heavy chain cDNA and light chain cDNA of 26B12H3L2, the heavy chain cDNA and light chain cDNA of 26B12H2L3, the heavy chain cDNA and light chain cDNA of 26B12H3L3, the heavy chain cDNA and light chain cDNA of 26B12H1L4, the heavy chain cDNA and light chain cDNA of 26B12H2L4, and the heavy chain cDNA and light chain cDNA of 26B12H4L4 were cloned into pUC57simple (provided by Genscript) vectors to obtain:
pUC57simple-26B12H1 and pUC57simple-26B12L1;
pUC57simple-26B12H4 and pUC57simple-26B12L1;
pUC57simple-26B12H2 and pUC57simple-26B12L2;
pUC57simple-26B12H3 and pUC57simple-26B12L2;
pUC57simple-26B12H2 and pUC57simple-26B12L3;
pUC57simple-26B12H3 and pUC57simple-26B12L3;
pUC57simple-26B12H1 and pUC57simple-26B12L4; and
pUC57simple-26B12H4 and pUC57simple-26B12L4, respectively.

With reference to the standard techniques described in Molecular Cloning: A Laboratory Manual (Second Edition), the heavy and light chain full-length genes synthesized by EcoRI & HindIII cleavage were subcloned into pcDNA3.1 expression vectors by cleavage with restriction enzymes EcoRI & HindIII to obtain expression plasmids pcDNA3.1-26B12H1, pcDNA3.1-26B12L1, pcDNA3.1-26B12H4, pcDNA3.1-26B12H2, pcDNA3.1-26B12L2, pcDNA3.1-26B12H3, pcDNA3.1-26B12L3, and pcDNA3.1-26B12L4, and the heavy/light chain genes of the recombinant expression plasmids were further sequenced. Subsequently, the designed gene combinations comprising corresponding light and heavy chain recombinant plasmids (pcDNA3.1-26B12H1/pcDNA3.1-26B12L1, pcDNA3.1-26B12H4/pcDNA3.1-26B12L1, pcDNA3.1-26B12H2/pcDNA3.1-26B12L2, pcDNA3.1-26B12H3/pcDNA3.1-26B12L2, pcDNA3.1-26B12H2/pcDNA3.1-26B12L3, pcDNA3.1-26B12H3/pcDNA3.1-26B12L3, pcDNA3.1-26B12H1/pcDNA3.1-26B12L4, and pcDNA3.1-26B12H4/pcDNA3.1-26B12L4) were separately co-transfected into 293F cells, and the culture solutions were then collected and purified. After the products were verified by sequencing, endotoxin-free expression plasmids were prepared and transiently transfected into HEK293 cells for antibody expression. After 7 days of culture, the cell culture solutions were collected and subjected to affinity purification on a Protein A column to obtain humanized antibodies.

Humanized antibody 26B12H2L2 is also referred to herein as 26B12H2L2(hG1WT).

### 3. Design of humanized antibodies 26B12H2L2(hG1DM) and 26B12H2L2(hG4DM)

On the basis of 26B12H2L2, the inventors introduced a leucine-to-alanine point mutation at position 234 (L234A) and a leucine-to-alanine point mutation at position 235 (L235A) in the heavy chain constant region thereof (according to the EU numbering system; the same applies hereinafter) to obtain a humanized antibody with the mutations in the constant region: 26B12H2L2(hG1DM). The amino acid sequence of the heavy chain of 26B12H2L2(hG1DM) is set forth in SEQ ID NO: 70, and the amino acid sequence of the light chain thereof is set forth in SEQ ID NO: 68.

On the basis of 26B12H2L2, with the variable regions unchanged, the inventors used Ig gamma-4 chain C region (ACCESSION: P01861.1) as the heavy chain constant region, and introduced a phenylalanine-to-alanine point mutation at position 234 (F234A) and a leucine-to-alanine point mutation at position 235 (L235A) in the heavy chain constant region to obtain a humanized antibody with the mutations in the constant region:
26B12H2L2(hG4DM). The amino acid sequence of the heavy chain of 26B12H2L2(hG4DM) is set forth in SEQ ID NO: 66, and the amino acid sequence of the light chain thereof is set forth in SEQ ID NO: 68.

Humanized antibodies 26B12H2L2(hG1DM) and 26B12H2L2(hG4DM) can be prepared with reference to the method described in step 2 above.

### Preparation Example 9: Sequence Design and Preparation of Anti-TIGIT Antibody-TGF-βR Fusion Proteins

### 1. Sequence design

The composition of the anti-TIGIT antibody-TGF-βR fusion proteins herein is shown in Table 3 below.

**Table 3: The composition of the heavy and light chains of the anti-TIGIT antibody-TGF-βR fusion proteins**

| **Fusion protein name** | **Heavy chain of IgG portion** | **Linker** | **Truncated fragment of TGF-βRII extracellular region** | **Light chain** |
|---|---|---|---|---|
| **TF01** | **SEQ ID NO: 66** | **SEQ ID NO: 83** | **SEQ ID NO: 72** | **SEQ ID NO: 68** |
| **TF02** | **SEQ ID NO: 70** | **SEQ ID NO: 83** | **SEQ ID NO: 72** | **SEQ ID NO: 68** |

The TGF-β receptor portion has two peptide chains, which are linked to the C-termini of the heavy chains of the IgG portion via linker fragments, respectively.

### 2. Expression and purification of antibodies

The heavy chain cDNA sequences of TF01 and TF02 and light chain cDNA sequences thereof were cloned into pUC57simple (provided by Genscript) vectors to obtain plasmids pUC57simple-TF01H and pUC57simple-TF02H, and pUC57simple-TF01L and pUC57simple-TF02L, respectively.

The plasmids pUC57simple-TF01H/pUC57simple TF01L and pUC57simple-TF02H/pUC57simple TF02L were subjected to enzyme cleavage (HindIII & EcoRI). The heavy and light chains were recovered by electrophoresis, and then each subcloned into the pcDNA3.1 vector. The recombinant plasmids were extracted and co-transfected into 293F cells. After 7 days of cell culture, the culture medium was subjected to high-speed centrifugation, and the supernatant was concentrated and then loaded onto a HiTrap MabSelect SuRe column. The protein was eluted in one step with an elution buffer. The target sample was recovered and buffer-exchanged into PBS. Thus, anti-TIGIT antibody-TGF-βR fusion proteins TF01 and TF02 were obtained.

### Preparation Example 10: Sequence Design and Preparation of Anti-TIGIT Antibody-TGF-βR Fusion Proteins (Mutants)

The TGF-βRII extracellular region fragment in the anti-TIGIT antibody-TGF-βR fusion protein TF01 of the present disclosure was subjected to point mutation to mutate serine at position 8 of the TGF-βRII extracellular region fragment (SEQ ID NO: 72) to threonine (SEQ ID NO: 88), and a new antibody was obtained, which was named TF01T. Reference was made to Preparation Example 9 for the preparation method therefor.

**Table 4: The composition of the heavy and light chains of the anti-TIGIT antibody-TGF-DR fusion proteins (mutants)**

| **Fusion protein name** | **Heavy chain of IgG portion** | **Linker** | **TGF-βRII extracellular region fragment variant** | **Light chain** |
|---|---|---|---|---|
| **TF01T** | **SEQ ID NO: 66** | **SEQ ID NO: 83** | **SEQ ID NO: 88** | **SEQ ID NO: 68** |

### Example 1: Promotion of INF-γ Secretion by Combination of Anti-TIGIT Antibody-TGF-βR Fusion Protein and Anti-PD-1-Anti-VEGFA Bispecific Antibody

Normal human PBMCs (isolated from healthy human peripheral blood) were obtained by separation according to the operating instructions of the separation solution Ficoll-Paque ^{™} Plus (Cytiva, Cat No. 17-1440-02). One day before the experiment, PBMCs were thawed and cultured in a complete medium (i.e., RPMI 1640 + 10% FBS; RPMI 1640, Gibco, Cat. No. 22400-089; FBS, fetal bovine serum, Excell Bio, Cat. No. FSP500) in a 5% carbon dioxide incubator at 37 °C. On the day of the experiment, the antibodies (VP101(hG1DM) and TF01T at a molar ratio of 1:1) serially diluted with a complete medium, CMV (Mabtech, Cat. No. 3619-1) and TGF-β1 (Genscript, Cat. No. Z03411) were grouped according to the experimental design. TGF-β1 (at a final concentration of 3 ng/mL) and the antibodies were added in equal volumes to a 96-well round-bottom plate (Corning, Model No. 3799). A control group was designed. The plate was incubated at 37 °C for 10 min. PBMCs were collected, counted, and determined for viability. The cells were seeded into the 96-well round-bottom plate at 200,000 cells/well, and CMV at a final concentration of 0.2 µg/mL was added. The plate was gently tapped on the edges for uniform mixing and then cultured in an incubator for 4 days (the final volume of the experimental system was 200 µL). After 4 days, the cell culture supernatant was collected, and the concentration of IFN-γ was determined using an ELISA kit (Dakewe, Cat. No. 1110002).

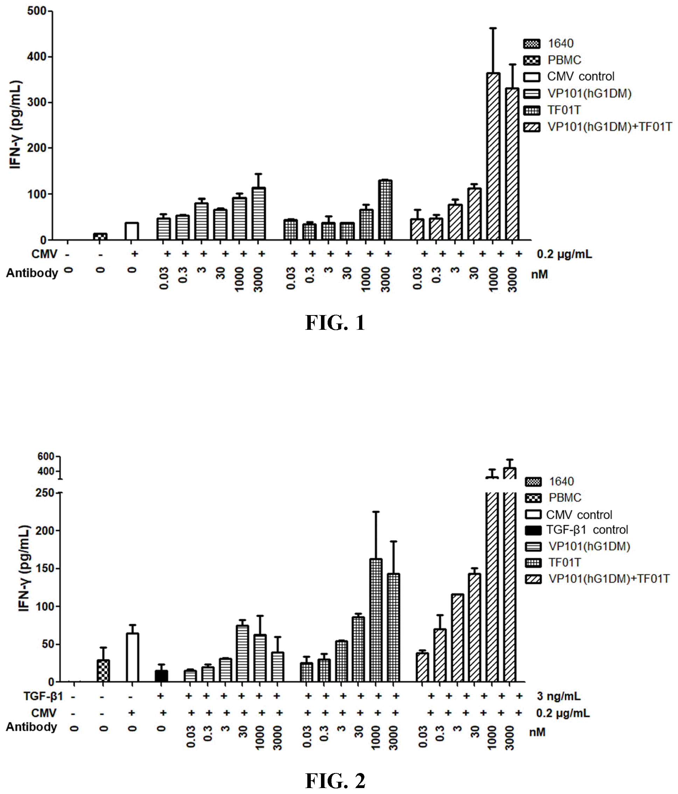

The results are shown in FIG. 1 and FIG. 2.

The results showed that under CMV stimulation, the VP101(hG1DM) monotherapy group, TF01T monotherapy group, and VP101(hG1DM) + TF01T group could all effectively promote the secretion of IFN-γ by PBMCs, and the ability of the VP101(hG1DM) + TF01T group to promote the secretion of IFN-γ by PBMCs was significantly stronger than that of the monotherapy groups. After TGF-β1 capable of inhibiting IFN-γ secretion in the system was added into the system, the IFN-γ secretion level of the VP101(hG1DM) + TF01T group was higher than that of the group without the addition of TGF-β1, showing stronger immunostimulatory biological activity of promoting PBMC recall response to CMV.

### Example 2: Verification of Blocking Activity of Combination of Anti-TIGIT Antibody-TGF-βR Fusion Protein and Anti-PD-1-Anti-VEGFA Bispecific Antibody by Reporter Gene System

PDL1 aAPC/CHO-K1 cells (purchased from Promega, Cat. No. J108A) were resuspended in Ham's F-12 + 10% FBS medium (Ham's F-12, Gibco, Cat. No. 11765070; FBS, fetal bovine serum, Excell Bio, Cat. No. FSP500), and then seeded into a 96-well flat-bottom black plate (Corning, Model No. 3916) at 40,000 cells/well/100 µL and cultured overnight. The next day, the antibody and VEGF (constructed in-house by Akeso Biopharma Inc., Cat. No. 20210508) were diluted with an assay medium (i.e., RPMI 1640 + 10% FBS; RPMI 1640, Gibco, Cat. No. 22400-089; FBS, fetal bovine serum, Excell Bio, Cat. No. FSP500) according to the experimental design, and the antibody and VEGF were pre-incubated at 37 °C for 30 min. Jurkat-NFAT-PD1-hTIGIT cells (constructed by Akeso Biopharma Inc.) and A549 cells (purchased from Cell Resource Center, Shanghai Institutes for Biological Sciences, Chinese Academy of Sciences, Cat. No. SCSP-503) were separately collected and resuspended in an assay medium, and the cell density was adjusted. The medium in the 96-well flat-bottom black plate was removed. According to the experimental design, the antibody or antibody-VEGF pre-incubation solution (VP101(hG1DM) and TF01T at a molar ratio of 1:1) was added at 40 µL/well, Jurkat-NFAT-PD1-hTIGIT cells were added at 50,000 cells/well/20 µL, and A549 cells were added at 10,000 cells/well/20 µL. An isotype control group (human IgG1DM) and a blank control group were set. The plate was incubated in an incubator for 6 h. The 96-well flat-bottom black plate was taken out and allowed to equilibrate to room temperature. Firefly Glo Luciferase Reporter Gene Assay Kit (Yeasen, Cat. No. 11404ES80) was added at 80 µL/well, and the mixture was incubated for 5 min in the dark. Then, the RLU values were read.

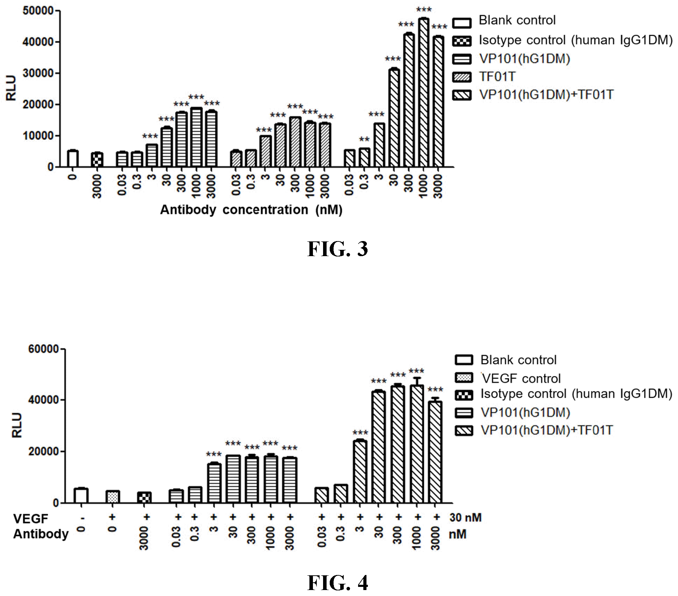

The results are shown in FIG. 3. In the reporter gene system, the TIGIT/PVR and PD-1/PD-L1 interactions can both inhibit the activation of the NFAT signaling pathway. Therefore, blocking TIGIT/PVR and/or PD-1/PD-L1 can activate the NFAT pathway in effector cells, thereby activating the expression of the luciferase reporter gene. The higher the expression of luciferase, the stronger the fluorescence intensity. The results showed that the VP101(hG1DM) monotherapy group, TF01T monotherapy, and VP101(hG1DM) + TF01T group could all effectively exert a neutralizing effect to block the TIGIT/PVR and PD-1/PD-L1 interactions, and the neutralizing biological activity of VP101(hG1DM) + TF01T in blocking TIGIT/PVR and PD-1/PD-L1 was superior to that of VP101(hG1DM) monotherapy and TF01T monotherapy.

In addition, VEGF was additionally added to the reporter gene system for pretreatment. The results are shown in FIG. 4. The results showed that compared with the isotype control human IgG1DM, the VP101(hG1DM) monotherapy group and VP101(hG1DM) + TF01Tgroup could both significantly improve luciferase expression; and the blocking activity of the VP101(hG1DM) + TF01T group was higher than that of the VP101(hG1DM) monotherapy group.

In conclusion, the neutralizing biological activity of VP101(hG1DM) + TF01T in blocking TIGIT/PVR and PD-1/PD-L1 is superior to that of VP101(hG1DM) monotherapy and TF01T monotherapy, and in the system containing VEGF, VP101(hG1DM) + TF01T shows stronger neutralizing activity.

Although the specific embodiments of the present disclosure have been described in detail, those skilled in the art will understand that various modifications and substitutions can be made to those details according to all the teachings that have been disclosed, and these changes shall all fall within the protection scope of the present disclosure. The full scope of the present disclosure is given by the appended claims and any equivalent thereof.

## Claims

1. A pharmaceutical combination comprising an effective amount of a first bispecific protein and an effective amount of a second bispecific protein, wherein
(1) the first bispecific protein comprises:
a first protein functional region specifically binding to PD-1, and
a second protein functional region specifically binding to VEGFA,
wherein
the first protein functional region comprises a heavy chain variable region VH1 and a light chain variable region VL1, wherein the VH1 comprises an HCDR1 set forth in SEQ ID NO: 1, an HCDR2 set forth in SEQ ID NO: 2, and an HCDR3 set forth in SEQ ID NO: 3; and the VL1 comprises an LCDR1 set forth in SEQ ID NO: 4, an LCDR2 set forth in SEQ ID NO: 5, and an LCDR3 set forth in SEQ ID NO: 6;
the second protein functional region comprises a heavy chain variable region VH2 and a light chain variable region VL2, wherein the VH2 comprises an HCDR1 set forth in SEQ ID NO: 7, an HCDR2 set forth in SEQ ID NO: 8, and an HCDR3 set forth in SEQ ID NO: 9; and the VL2 comprises an LCDR1 set forth in SEQ ID NO: 10, an LCDR2 set forth in SEQ ID NO: 11, and an LCDR3 set forth in SEQ ID NO: 12;
(2) the second bispecific protein comprises:
a third protein functional region specifically binding to TIGIT, and
a fourth protein functional region specifically binding to TGF-β,
wherein
the third protein functional region comprises a heavy chain variable region VH3 and a light chain variable region VL3, wherein the VH3 comprises an HCDR1 set forth in SEQ ID NO: 13, an HCDR2 set forth in SEQ ID NO: 14, and an HCDR3 set forth in SEQ ID NO: 15; and the VL3 comprises an LCDR1 set forth in SEQ ID NO: 16, an LCDR2 set forth in SEQ ID NO: 17, and an LCDR3 set forth in SEQ ID NO: 18;
the fourth protein functional region is a TGF-β receptor, an extracellular region of a TGF-β receptor, a truncated fragment of an extracellular region of a TGF-β receptor with TGF-β receptor function, or a variant of an extracellular region of a TGF-β receptor with TGF-β receptor function.

2. The pharmaceutical combination according to claim 1, wherein
the heavy chain variable region VH1 comprises an amino acid sequence selected from SEQ ID NO: 20 and SEQ ID NO: 24; and
the light chain variable region VL1 comprises an amino acid sequence selected from SEQ ID NO: 22, SEQ ID NO: 26, and SEQ ID NO: 35.

3. The pharmaceutical combination according to any one of claims 1 to 2, wherein
the heavy chain variable region VH1 comprises an amino acid sequence set forth in SEQ ID NO: 20, and the light chain variable region VL1 comprises an amino acid sequence set forth in SEQ ID NO: 22;
the heavy chain variable region VH1 comprises an amino acid sequence set forth in SEQ ID NO: 20, and the light chain variable region VL1 comprises an amino acid sequence set forth in SEQ ID NO: 26;
the heavy chain variable region VH1 comprises an amino acid sequence set forth in SEQ ID NO: 20, and the light chain variable region VL1 comprises an amino acid sequence set forth in SEQ ID NO: 35;
the heavy chain variable region VH1 comprises an amino acid sequence set forth in SEQ ID NO: 24, and the light chain variable region VL1 comprises an amino acid sequence set forth in SEQ ID NO: 22;
the heavy chain variable region VH1 comprises an amino acid sequence set forth in SEQ ID NO: 24, and the light chain variable region VL1 comprises an amino acid sequence set forth in SEQ ID NO: 26; or
the heavy chain variable region VH1 comprises an amino acid sequence set forth in SEQ ID NO: 24, and the light chain variable region VL1 comprises an amino acid sequence set forth in SEQ ID NO: 35.

4. The pharmaceutical combination according to any one of claims 1 to 3, wherein
the heavy chain variable region VH2 comprises an amino acid sequence set forth in SEQ ID NO: 31, and the light chain variable region VL2 comprises an amino acid sequence set forth in SEQ ID NO: 33.

5. The pharmaceutical combination according to any one of claims 1 to 4, wherein
the heavy chain variable region VH3 comprises an amino acid sequence selected from SEQ ID NO: 46, SEQ ID NO: 50, SEQ ID NO: 52, SEQ ID NO: 54, and SEQ ID NO: 56; and
the light chain variable region VL3 comprises an amino acid sequence selected from SEQ ID NO: 48, SEQ ID NO: 58, SEQ ID NO: 60, SEQ ID NO: 62, and SEQ ID NO: 64.

6. The pharmaceutical combination according to any one of claims 1 to 5, wherein
the heavy chain variable region VH3 comprises an amino acid sequence set forth in SEQ ID NO: 46, and the light chain variable region VL3 comprises an amino acid sequence set forth in SEQ ID NO: 48;
the heavy chain variable region VH3 comprises an amino acid sequence set forth in SEQ ID NO: 50, and the light chain variable region VL3 comprises an amino acid sequence set forth in SEQ ID NO: 58;
the heavy chain variable region VH3 comprises an amino acid sequence set forth in SEQ ID NO: 50, and the light chain variable region VL3 comprises an amino acid sequence set forth in SEQ ID NO: 64;
the heavy chain variable region VH3 comprises an amino acid sequence set forth in SEQ ID NO: 52, and the light chain variable region VL3 comprises an amino acid sequence set forth in SEQ ID NO: 60;
the heavy chain variable region VH3 comprises an amino acid sequence set forth in SEQ ID NO: 52, and the light chain variable region VL3 comprises an amino acid sequence set forth in SEQ ID NO: 62;
the heavy chain variable region VH3 comprises an amino acid sequence set forth in SEQ ID NO: 54, and the light chain variable region VL3 comprises an amino acid sequence set forth in SEQ ID NO: 60;
the heavy chain variable region VH3 comprises an amino acid sequence set forth in SEQ ID NO: 54, and the light chain variable region VL3 comprises an amino acid sequence set forth in SEQ ID NO: 62;
the heavy chain variable region VH3 comprises an amino acid sequence set forth in SEQ ID NO: 56, and the light chain variable region VL3 comprises an amino acid sequence set forth in SEQ ID NO: 58; or
the heavy chain variable region VH3 comprises an amino acid sequence set forth in SEQ ID NO: 56, and the light chain variable region VL3 comprises an amino acid sequence set forth in SEQ ID NO: 64.

7. The pharmaceutical combination according to any one of claims 1 to 6, wherein
the fourth protein functional region is TGF-βRI, TGF-βRII, TGF-βRIII, an extracellular region of TGF-βRI, an extracellular region of TGF-βRII, an extracellular region of TGF-βRIII, a truncated fragment of an extracellular region of TGF-βRI with TGF-β receptor function, a truncated fragment of an extracellular region of TGF-βRII with a TGF-β receptor function, a truncated fragment of an extracellular region of TGF-βRIII with a TGF-β receptor function, or a variant of an extracellular region of TGF-β receptor with a TGF-β receptor function.

8. The pharmaceutical combination according to any one of claims 1 to 7, wherein
the first protein functional region is IgG, and the second protein functional region is a single-chain variable fragment; or
the first protein functional region is a single-chain variable fragment, and the second protein functional region is IgG.

9. The pharmaceutical combination according to any one of claims 1 to 8, wherein
the third protein functional region is IgG or a single-chain variable fragment.

10. The pharmaceutical combination according to any one of claims 8 to 9, wherein
the IgG is IgG1, IgG2, IgG3, or IgG4.

11. The pharmaceutical combination according to any one of claims 8 to 10, wherein
according to the EU numbering system, the heavy chain constant region of IgG1 has one of the following four combinations of mutations:
L234A and L235A;
L234A and G237A;
L235A and G237A; or
L234A, L235A, and G237A.

12. The pharmaceutical combination according to any one of claims 8 to 10, wherein
according to the EU numbering system, the heavy chain constant region of IgG4 has one of the following four combinations of mutations:
F234A and L235A;
F234A and G237A;
L235A and G237A; or
F234A, L235A, and G237A.

13. The pharmaceutical combination according to any one of claims 1 to 12, wherein
the second protein functional region is IgG, the heavy chain thereof comprises an amino acid sequence set forth in SEQ ID NO: 38, SEQ ID NO: 40, or SEQ ID NO: 42, and the light chain thereof comprises an amino acid sequence set forth in SEQ ID NO: 44.

14. The pharmaceutical combination according to any one of claims 1 to 13, wherein
the third protein functional region is IgG, the heavy chain comprises an amino acid sequence set forth in SEQ ID NO: 66 or SEQ ID NO: 70, and the light chain comprises an amino acid sequence set forth in SEQ ID NO: 68.

15. The pharmaceutical combination according to any one of claims 1 to 14, wherein
the fourth protein functional region comprises an amino acid sequence set forth in any one of SEQ ID NO: 72 to SEQ ID NO: 78 and SEQ ID NO: 88.

16. The pharmaceutical combination according to any one of claims 1 to 15, wherein
the first protein functional region and the second protein functional region are linked directly or via a linker;
the third protein functional region and the fourth protein functional region are linked directly or via a linker; and/or
a heavy chain variable region of a single-chain variable fragment and a light chain variable region of a single-chain variable fragment are linked directly or via a linker.

17. The pharmaceutical combination according to claim 16, wherein
the linker is a polypeptide set forth in SEQ ID NO: 91 or SEQ ID NO: 92, or a polypeptide obtained by concatenating a plurality of (e.g., 2, 3, 4, 5, or 6) polypeptides set forth in SEQ ID NO: 91, or a polypeptide obtained by concatenating a plurality of (e.g., 2, 3, 4, or 5) polypeptides set forth in SEQ ID NO: 91 and further concatenating the polypeptides with a polypeptide set forth in SEQ ID NO: 92;
preferably, the linker comprises an amino acid sequence independently selected from SEQ ID NO: 36, SEQ ID NO: 37, and SEQ ID NO: 83.

18. The pharmaceutical combination according to any one of claims 1 to 17, wherein
the first protein functional region, the second protein functional region, the third protein functional region, and the fourth protein functional region are independently 1, 2 or more in number;
preferably, the first protein functional region is 2 in number, and the second protein functional region is 1 in number;
preferably, the third protein functional region is 1 in number, and the fourth protein functional region is 2 in number.

19. The pharmaceutical combination according to any one of claims 1 to 18, wherein
the second protein functional region is one IgG (e.g., IgG1), the first protein functional region is two single-chain variable fragments, and the single-chain variable fragments are linked to the C-termini or the N-termini of the two heavy chains of the second protein functional region, respectively.

20. The pharmaceutical combination according to any one of claims 1 to 19, wherein
the third protein functional region is one IgG (e.g., IgG4), the fourth protein functional region is 2 in number, and the fourth protein functional regions are linked to the C-termini or the N-termini of the two heavy chains of the third protein functional region, respectively.

21. The pharmaceutical combination according to any one of claims 1 to 20, wherein
the first bispecific protein comprises:
a first protein functional region specifically binding to PD-1, and
a second protein functional region specifically binding to VEGFA;
the first protein functional region is 2 in number, and the second protein functional region is 1 in number,
wherein the second protein functional region is IgG, and the first protein functional region is a single-chain variable fragment;
the IgG comprises a heavy chain having an amino acid sequence set forth in SEQ ID NO: 42, and a light chain having an amino acid sequence set forth in SEQ ID NO: 44;
the single-chain variable fragment comprises a heavy chain variable region having an amino acid sequence set forth in SEQ ID NO: 24, and a light chain variable region having an amino acid sequence set forth in SEQ ID NO: 35;
two single-chain variable fragments are linked to the C-termini of the two heavy chains of the IgG, respectively;
the first protein functional region and the second protein functional region are linked via a first linker; the heavy chain variable region of the single-chain variable fragment and the light chain variable region of the single-chain variable fragment are linked via a second linker; the first linker and the second linker are identical or different;
preferably, the first linker and the second linker each comprise an amino acid sequence independently selected from SEQ ID NO: 36 and SEQ ID NO: 37;
preferably, the first linker and the second linker both comprise an amino acid sequence set forth in SEQ ID NO: 36.

22. The pharmaceutical combination according to any one of claims 1 to 21, wherein
the second bispecific protein comprises:
a third protein functional region specifically binding to TIGIT, and
a fourth protein functional region specifically binding to TGF-β,
wherein
the third protein functional region is 1 in number, and the fourth protein functional region is 2 in number;
the third protein functional region is IgG, and the fourth protein functional region is a truncated fragment of an extracellular region of a TGF-β receptor with TGF-β receptor function;
the IgG comprises a heavy chain having an amino acid sequence set forth in SEQ ID NO: 66 or SEQ ID NO: 70, and a light chain having an amino acid sequence set forth in SEQ ID NO: 68;
the fourth protein functional region comprises an amino acid sequence set forth in SEQ ID NO: 72 or SEQ ID NO: 88;
the fourth protein functional regions are linked to the C-termini of the two heavy chains of the IgG via a third linker;
preferably, the third linker comprises an amino acid sequence set forth in SEQ ID NO: 83.

23. The pharmaceutical combination according to any one of claims 1 to 22, wherein the first bispecific protein and the second bispecific protein are mixed together, and the pharmaceutical combination is a pharmaceutical composition.

24. The pharmaceutical combination according to any one of claims 1 to 22, wherein the first bispecific protein and the second bispecific protein are in separate packages, and the pharmaceutical combination is a combination product.

25. The pharmaceutical combination according to any one of claims 1 to 24, wherein a mass ratio of the first bispecific protein to the second bispecific protein is (10:1) to (1:5), (5:1) to (1:3), (3:1) to (1:2), or 2:1.

26. The pharmaceutical combination according to any one of claims 1 to 24, wherein a molar ratio of the first bispecific protein to the second bispecific protein is (10:1) to (1:10), (5:1) to (1:5), (3:1) to (1:3), (2:1) to (1:2), or 1:1.

27. The pharmaceutical combination according to any one of claims 1 to 26, wherein unit doses of the first bispecific protein and the second bispecific protein are independently 100 mg to 2500 mg, 100 mg to 2000 mg, 100 mg to 1500 mg, 100 mg to 1200 mg, 100 mg to 1000 mg, 200 mg to 800 mg, 200 mg to 500 mg, 300 mg to 600 mg, 400 mg to 500 mg, or 450 mg, based on the mass of the first bispecific protein or the second bispecific protein.

28. The pharmaceutical combination according to any one of claims 1 to 27, wherein the pharmaceutical combination further comprises an effective amount of an anti-tumor chemotherapeutic drug, such as a platinum-based drug, an alkylating agent, an antimetabolite, an anti-tumor antibiotic, a plant-based anticancer agent, a hormone, or an immunological agent.

29. The pharmaceutical combination according to any one of claims 1 to 28, wherein the pharmaceutical combination further comprises one or more pharmaceutically acceptable auxiliary materials; preferably, the pharmaceutical combination further comprises a package insert.

30. Use of the pharmaceutical combination according to any one of claims 1 to 29 in the preparation of a medicament for treating or preventing a tumor, wherein
preferably, the tumor is selected from one or more of colon cancer, rectal cancer, lung cancer, liver cancer, ovarian cancer, skin cancer, glioma, melanoma, lymphoma, renal tumor, prostate cancer, bladder cancer, gastrointestinal cancer, breast cancer, brain cancer, cervical cancer, esophageal cancer, urothelial carcinoma, mesothelioma, endometrial cancer, gastric adenocarcinoma, gastroesophageal junction adenocarcinoma, leukemia, multiple myeloma, plasma cell cancer, and pancreatic cancer;
preferably, the lung cancer is non-small cell lung cancer or small cell lung cancer;
preferably, the liver cancer is hepatocellular carcinoma;
preferably, the renal cancer is renal cell carcinoma;
preferably, the breast cancer is triple negative breast cancer;
preferably, the urothelial carcinoma is bladder cancer;
preferably, the lymphoma is non-Hodgkin's lymphoma or B-lymphoma.

31. The pharmaceutical combination according to any one of claims 1 to 29 for use in the treatment or prevention of a tumor, wherein
preferably, the tumor is selected from one or more of colon cancer, rectal cancer, lung cancer, liver cancer, ovarian cancer, skin cancer, glioma, melanoma, lymphoma, renal tumor, prostate cancer, bladder cancer, gastrointestinal cancer, breast cancer, brain cancer, cervical cancer, esophageal cancer, urothelial carcinoma, mesothelioma, endometrial cancer, gastric adenocarcinoma, gastroesophageal junction adenocarcinoma, leukemia, multiple myeloma, plasma cell cancer, and pancreatic cancer;
preferably, the lung cancer is non-small cell lung cancer or small cell lung cancer;
preferably, the liver cancer is hepatocellular carcinoma;
preferably, the renal cancer is renal cell carcinoma;
preferably, the breast cancer is triple negative breast cancer;
preferably, the urothelial carcinoma is bladder cancer;
preferably, the lymphoma is non-Hodgkin's lymphoma or B-lymphoma.

32. A method for treating or preventing a tumor, comprising a step of administering to a subject in need an effective amount of the pharmaceutical combination according to any one of claims 1 to 29, wherein
preferably, the tumor is selected from one or more of colon cancer, rectal cancer, lung cancer, liver cancer, ovarian cancer, skin cancer, glioma, melanoma, lymphoma, renal tumor, prostate cancer, bladder cancer, gastrointestinal cancer, breast cancer, brain cancer, cervical cancer, esophageal cancer, urothelial carcinoma, mesothelioma, endometrial cancer, gastric adenocarcinoma, gastroesophageal junction adenocarcinoma, leukemia, multiple myeloma, plasma cell cancer, and pancreatic cancer;
preferably, the lung cancer is non-small cell lung cancer or small cell lung cancer;
preferably, the liver cancer is hepatocellular carcinoma;
preferably, the renal cancer is renal cell carcinoma;
preferably, the breast cancer is triple negative breast cancer;
preferably, the urothelial carcinoma is bladder cancer;
preferably, the lymphoma is non-Hodgkin's lymphoma or B-lymphoma.

33. The method according to claim 32, wherein the first bispecific protein and the second bispecific protein are administered independently before or after surgery and/or before or after radiotherapy.

34. The method according to any one of claims 32 to 33, wherein
the first bispecific protein and the second bispecific protein are independently administered at a single dose of 0.1-100 mg, 5-50 mg, or 5-15 mg per kg body weight;
the first bispecific protein and the second bispecific protein are independently administered once every half day, 1 day, 2 days, 3 days, 4 days, 5 days, 6 days, 10 days, 1 week, 2 weeks, or 3 weeks;
and/or
the administration is performed via a route of intravenous drip infusion, intravenous injection, subcutaneous injection, or intraperitoneal injection.
